# EUROPEAN PATENT SPECIFICATION

(11) **EP 4 312 818 B1**
(45) Date of publication and mention of the grant of the patent: **04.03.2026**
(21) Application number: 22776484.2
(22) Date of filing: 22.03.2022
(51) Int. Cl.: A61B 17/11

(54) **LAPAROSCOPIC ANASTOMOSIS DEVICES**
LAPAROSKOPISCHE ANASTOMOSEVORRICHTUNGEN
DISPOSITIFS D'ANASTOMOSE LAPAROSCOPIQUE

(30) Priority: 22.03.2021 US 202163164493 P
(43) Date of publication of application: 07.02.2024
(73) Proprietor: Vivifi Medical, Inc., Rosharon, Texas 77583 (US)
(72) Inventor: SHARMA, Tushar, Rosharon, Texas 77583 (US); GARBIN, Nicolo, Houston, Texas 77005 (US); PARDO, Juan, Houston, Texas 77046 (US)
(74) Representative: Cooley (UK) LLP
(86) International application number: PCT/US2022/021363
(87) International publication number: WO 2022/204160

(56) References cited:
- US-A- 3 048 177
- US-A- 3 316 914
- US-A- 4 245 638
- US-A1- 2005 251 179
- US-A1- 2011 251 628
- US-A1- 2011 319 916
- US-A1- 2020 178 966

## Description

### CROSS-REFERENCE TO RELATED APPLICATIONS

This application claims priority to and the benefit of U.S. Provisional Application No. 63/164,493, filed March 22, 2021, and entitled "Devices and Methods for Anastomosis".

### STATEMENT OF GOVERNMENT SUPPORT

This invention was made with government support under Contract No. 2026272 awarded by the National Science Foundation. The government has certain rights in the invention.

### TECHNICAL FIELD

The embodiments described herein relate generally to apparatus for laparoscopically performing anastomosis of two tubular structures.

### BACKGROUND

An anastomosis is a connection between two luminal structures. Commonly, anastomotic connections are surgically created on blood vessels (such as veins or arteries), or tubular gastrointestinal structures (such as the intestines). Conventional techniques allow the anastomosis to be completed between two ends (referred to as end-to-end anastomosis), or between the end of one structure and the side of another structure (referred to as end-to-side anastomosis). Procedures requiring these anastomoses are carried out thousands of times per day, globally. Likewise, multiple surgical specialties rely upon the creation of reliable, unobstructed anastomoses for successful treatment of their respective patients. The surgical reattachment of veins and arteries (occasionally referred to herein for the sake of brevity as anastomosis ) helps to restore blood circulation, and consequently, improves the supply of oxygen and other nutrients to downstream tissues as well as improve the return of deoxygenated blood from tissues back into the circulatory system. Thus, it is desirable to perform various vessel anastomosis procedures reliably using minimally invasive procedures and with reduced complexity.

US 2011/319916 A1 discloses an anastomosis device comprising two holders configured for releasably holding a respective anastomosis ring, wherein the holders are disposed such that the anastomosis rings are spaced appart from each other at a standby position, are connected to each other in a second position. For this purpose, the anastomosis device comprises two slave and corresponding guide bars, to which the holders are connected, and a driver, slidably disposed over the guide bars, and configured to urge the guide bars towards the second position when the guide bars is axially moved in relation to the guide bars.

US 3316914 A discloses a device for joining vessel ends comprising two arms for holding a corresponding split sleeve and ring configured to connect two ends of a vessel. The arms are moved from a first position, spaced appart from each other, into a second position, in which the split sleeves and rings are connected to each other by a system of links connected to a reciprocating bar which is configured to axially move in order to urge the arms towards the first or the second position.

US 2011/251628 A1 discloses an anastomotic device comprising two holders configured to hold and connect a respective anastomotic ring. Each holder comprises a slave body comprising a respective slave inclination surface configured to urge the holder towards each other, for connecting the anastomotic rings, when the inclined surfaces are axially retracted towards two corresponding driving bodies, each comprising a drive inclination surface.

### SUMMARY

Embodiments described herein relate to microsurgical instruments, minimally invasive surgery, and laparoscopic surgical devices. More specifically, embodiments described herein relate to systems and devices for performing anastomosis of tubular structures via a minimally invasive or laparoscopic surgical approach. Embodiments described herein additionally relate to laparoscopic systems and devices intended for use in-conjunction with joinable rings or other anastomotic couplers used in microvascular anastomosis to facilitate the end-to-end or end-to-side coaptation of vascular structures (such as arteries and/or veins).

In some embodiments, an apparatus, includes an interface, and an elongate body coupled to the interface and extending longitudinally therefrom. An end effector is coupled to a distal end of the elongate body. The end effector includes first end effector element defining a first receptacle structured to hold a first coupler element of a coupler, the first coupler element configured to receive an axial end of a first vessel, and a second end effector element defining a second receptacle structured to hold a second coupler element of the coupler, the second coupler element configured to receive an axial end of a second vessel. The end effector is configured to move between a first configuration in which at least a portion of the first end effector element is separate from a corresponding portion of the second end effector element such that the first coupler element is separate from the second coupler element, and a second configuration in which at least the portion of first end effector element is proximate to the corresponding portion of the second end effector element such that the first coupler element is coupled to the second coupler element so as to couple the first vessel to the second vessel.

**In** some embodiments, an apparatus includes an elongate body, and an end effector coupled to a distal end the elongate body. The end effector includes a first end effector element defining a first receptacle structured to hold a first coupler element of a coupler, the first coupler element configured to receive an axial end of a first vessel, and a second end effector element defining a second receptacle structured to hold a second coupler element of the coupler, the second coupler element configured to receive an axial end of a second vessel. A securement mechanism is operatively coupled to the end effector and configured to secure at least one of the first coupler element or the second coupler element in a first configuration of the end effector. The end effector is configured to move between the first configuration in which at least a portion of the first end effector element is separate from a corresponding portion of the second end effector element such that the first coupler element is separate from the second coupler element, and a second configuration in which at least the portion of first end effector element is proximate to the corresponding portion of the second end effector element such that the first coupler element is coupled to the second coupler element so as to couple the first vessel to the second vessel.

In some embodiments (not falling within the scope of the claims), a method is provided for anastomosis of a first vessel with a second vessel within a body of a patient via an apparatus including an interface, an elongate body coupled to the interface, and an end effector including a first end effector element and a second end effector element. The method includes inserting a distal end of the elongate body into the body of the patient. The end effector is moved into a first configuration in which at least a portion of the first end effector element is separate from a corresponding portion of the second end effector element such that a first coupler element coupled to the first end effector element is separate from a second coupler element coupled to the second end effector element. An axial end of the first vessel is inserted through the first coupler element. An axial end of the second vessel is inserted through the second coupler element. The end effector is actuated to cause at least the portion of first end effector element to move proximate to the corresponding portion of the second end effector element such that the first coupler element is coupled to the second coupler element, thereby coupling the first vessel to the second vessel. In some embodiments, the method may also include releasing the first coupler element from the first end effector element and the second coupler element from the second end effector element, and withdrawing the elongate body from the body of the patient.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

### BRIEF DESCRIPTION OF DRAWINGS

The foregoing and other features of the present disclosure will become more fully apparent from the following description and appended claims, taken in conjunction with the accompanying drawings. Understanding that these drawings depict only several implementations in accordance with the disclosure and are therefore, not to be considered limiting of its scope, the disclosure will be described with additional specificity and detail through use of the accompanying drawings.
FIG. 1A is a schematic illustration of an apparatus for performing laparoscopic anastomosis of two vessels, and FIG. 1B is schematic illustration of a distal portion of the apparatus of FIG. 1A illustrating a first vessel and a second vessel being coupled to each other via the apparatus of FIG. 1A, according to an embodiment.
FIG. 2 is a schematic flow chart of a method for laparoscopically coupling a first vessel to a second vessel, according to an embodiment.
FIG. 3 is a side perspective view of an anastomosis apparatus with an end effector of the apparatus, and an enlarged view of the end effector in one configuration, according to an embodiment.

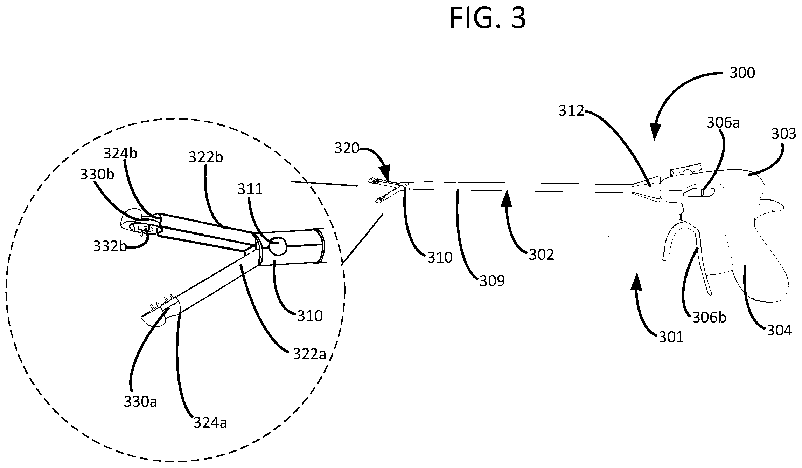

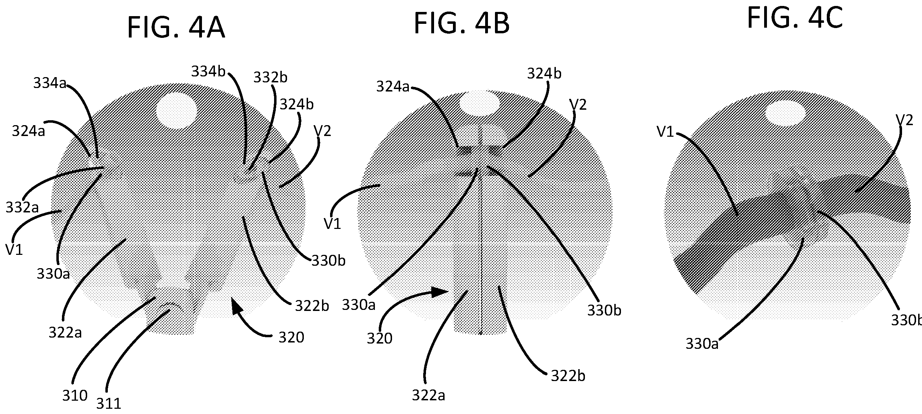

FIG. 4A is a top perspective view of the end effector of FIG. 3 in a first configuration within a body of a patient with a first vessel inserted through a first coupler element of a coupler coupled to a first end effector element, and a second vessel inserted through a second coupler element of the coupler coupled to a second coupler element of the coupler. FIG. 4B is a top perspective view of the end effector in a second configuration in which the first coupler element is coupled to the second coupler element to couple the first vessel to the second vessel. FIG. 4C shows the apparatus being removed from the coupler leaving the coupler within the body of the patient and first vessel being coupled to the second vessel.
FIG. 5A shows a side perspective view and a side view of a portion of an anastomosis apparatus in a first (open) configuration, according to an embodiment. FIG. 5B is a side perspective view and a side view of the portion of the apparatus of FIG. 5A in a third or intermediate (delivery) configuration. FIG. 5C is a side perspective view and a side view of the portion of the apparatus of FIG. 5A in a second (closed) configuration.
FIG. 6A is a top view and FIG. 6B is a side view of a portion of an anastomosis apparatus in a second (closed) configuration, according to an embodiment. FIG. 6C is a top view and FIG. 6D is a side view of the portion of the apparatus in a first (open) configuration. FIG. 6E is a schematic illustration of a trajectory of an end effector of the apparatus of FIGS. 6A-6D between the first and the second configurations.
FIG. 7A is a front view, FIG. 7B is a side perspective view, and FIG. 7C is a top view of a portion of an anastomosis apparatus in a second (closed) configuration, according to an embodiment. FIG. 7D is a top view and FIG. 7E is a side perspective view of the portion of the apparatus in a first (open) configuration.
FIG. 8A is a top view and FIG. 8B is a front view of an apparatus that includes resilient elements and closure elements, according to an embodiment.
FIG. 9A is top view of a portion of an anastomosis apparatus that includes an end effector including a first end effector element and a second end effector element that are configured to bend along a longitudinal axis thereof, in a second (closed) configuration, and FIG. 9B is a top view of the apparatus in a first (open) configuration, according to an embodiment.
FIG. 10A is a top view and FIG. 10B is a side view of an anastomosis apparatus including a biasing member in a second (closed) configuration, according to an embodiment. FIG. 10C is a top view and FIG. 10D is a side view of the apparatus of FIG. 10A is a first (open) configuration.
FIG. 11 is a side view of an end effector that may be included in an anastomosis apparatus, according to an embodiment.
FIG. 12A is a side view of a portion of an anastomosis apparatus that includes a securement mechanism, and FIG. 12B is a side perspective view of a first coupler element that the securement mechanism may secure, according to an embodiment.
FIG. 13A is a side view of a portion of an anastomosis apparatus that includes a securement mechanism, and FIG. 13B is a side perspective view of a first coupler element that the securement mechanism may secure, according to an embodiment.
FIG. 14A is a side view of a portion of an anastomosis apparatus that includes a release mechanism in a first configuration, according to an embodiment. FIG. 14B is a side view of the portion of the anastomosis apparatus with the release mechanism in a second configuration, according to an embodiment.
FIG. 15 is a schematic flow chart of a method for coupling a first vessel to a second vessel using an anastomosis apparatus, according to an embodiment.
FIG. 16 is a schematic flow chart of a method for coupling a first vessel to a second vessel using an anastomosis apparatus, according to an embodiment.
FIG. 17 are schematic illustrations of various steps of a method for coupling first vessel to a second vessel using an anastomosis apparatus, according to an embodiment.

Reference is made to the accompanying drawings throughout the following detailed description. In the drawings, similar symbols typically identify similar components, unless context dictates otherwise. The illustrative implementations described in the detailed description, drawings, and claims are not meant to be limiting. Other implementations may be utilized, and other changes may be made, without departing from the scope of the subject matter presented here. It will be readily understood that the aspects of the present disclosure, as generally described herein, and illustrated in the figures, can be arranged, substituted, combined, and designed in a wide variety of different configurations, all of which are explicitly contemplated and made part of this disclosure.

### DETAILED DESCRIPTION

Embodiments described herein relate to microsurgical instruments, minimally invasive surgery, and laparoscopic surgical devices. More specifically, embodiments described herein relate to systems, methods, and devices for performing anastomosis of tubular structures via a minimally invasive or laparoscopic surgical approach. Embodiments described herein additionally relate to laparoscopic systems, methods, and devices intended for use in-conjunction with joinable rings or other anastomotic couplers used in microvascular anastomosis to facilitate the end-to-end coaptation of vascular structures (such as arteries and/or veins).

An anastomosis is a connection between two luminal structures. Commonly, anastomotic connections are surgically created on blood vessels (such as veins or arteries), or tubular gastrointestinal structures (such as the intestines). Conventional techniques allow the anastomosis to be completed between two ends (referred to as end-to-end anastomosis), or between the end of one structure and the side of another structure (referred to as end-to-side anastomosis). Procedures requiring these anastomoses are carried out thousands of times per day, globally. Likewise, multiple surgical specialties rely upon the creation of reliable, unobstructed anastomoses for successful treatment of their respective patients. The surgical reattachment of veins and helps to restore blood circulation, and consequently, improves the supply of oxygen and other nutrients to downstream tissues as well as improve the return of deoxygenated blood from tissues back into the circulatory system. It is desirable to perform various vessel anastomosis procedures using minimally invasive procedures and with reduced complexity.

The original technique for performing an anastomosis was created by Alexis Carrel, who was later awarded the Nobel Prize in 1912 for his pioneering work. Despite 110 years of surgical evolution and innovation since the development of microsurgical anastomosis, the majority of vascular anastomoses to this day still employ suture techniques similar to Carrel's initial description in the early 1900s. In the 1970s, gastrointestinal stapling devices were introduced, which quickly replaced primary suture techniques for bowel anastomoses. However, most surgeons still employ circumferential suture techniques in the serosal layer overlying the stapled anastomosis for added support. Although generally successful, these techniques can take long periods of time, often require additional surgical expertise, and if not performed correctly, may result in leakage (blood, stool contents, gastric contents, lymphatic fluid, etc.), constriction, stenosis, and/or obstruction at the anastomotic site. In the case of vascular anastomoses, stenosis and/or obstruction can result in catastrophic complications such as heart attack, stroke, peripheral limb ischemia, amputation, death, and reconstructive failure and soft-tissue loss.

With the understood importance of reliable, open anastomoses, microvascular anastomotic couplers can provide superior alternatives to sutures and staples. Microvascular anastomotic couplers can consist of two coupler circular rings, each with a tissue-engaging surface comprising a multitude of sharp spikes. A blood vessel is brought through the center of each ring and the vessel wall is everted, or rolled over, the tissue spikes for securement. This is completed on each vessel end, and the two rings are then brought together with the spikes/pins being forced into the opposite ring to join the ends together.

Microvascular anastomosis of blood vessels can be accomplished using an anastomotic coupling device, such as the GEM^{™} FLOW COUPLER device. Current anastomotic couplers, however, are not designed or easily adaptable for use in minimally invasive procedures such as laparoscopy or microsurgery due to poor visibility and tight operating spaces as well as the lack of a device for applying two anastomotic couplers together that can be introduced through the small diameter of a trocar port.

Due to the lack of a reliable device or technique to apply anastomotic couplers laparoscopically, manual suturing is predominantly used for surgical coaptation of blood vessels in minimally invasive procedures. Manual suturing of blood vessels can be quite challenging, primarily due to the small size of the vessels and the minimal working space. Since most vessels are only 1 mm to 8 mm in diameter, the procedure generally includes the use of a surgical microscope. The sutures are about 70 µm thick and can be difficult to handle. As a result, surgeons and surgical residents must undergo extensive additional training prior to operating on a patient in need of tissue transfer. Moreover, surgeons attempt to limit the recipient site morbidity resulting in small incisions and small areas within which to work. For instance, in microsurgical postmastectomy breast reconstruction, the surgeon will typically be working in a 2.5 cm to 3 cm surgical field. These size constraints make it difficult for surgeons to maneuver their surgical instruments. Arterial anastomosis performed by manual suturing take approximately 23.5 minutes in the operating room, which is undesirable.

In contrast, embodiments of the anastomosis apparatus and methods of use described herein may provide one or more benefits, including, for example: 1) allowing minimally invasive laparoscopic surgery for performing anastomosis of two tubular tissues, for example, vessels, nerves, etc., through a small incision having a diameter of less than about 25 mm; 2) enabling anastomosis by simple clamping of coupler elements via an end effector that includes a pair of end effector elements configured to separate or close to couple the coupler elements, allowing anastomosis of two vessels within a time of about 5 minutes or less; 4) enabling articulation of the end effector about a longitudinal axis to provide surgical flexibility and allowing the end effector to access hard to reach areas; 5) providing an elongate body to which the end effector is coupled, which may have a length greater than about 8 inches (20.32 cm) allowing insertion through an incision in the abdominal wall while still allowing anastomosis of vessels in the pelvic floor of a patient; 6) providing a multi-functional interface that allows performing of all anastomosis related operations from the interface, thus reducing the use of manual handling with tweezers; and 7) providing significant clinical applicability, utility, and novelty in various surgical procedures for the treatment of numerous pathophysiological disorders, urological conditions, chronic diseases, and clinical indications including, but not limited to, varicocele repair, cardiovascular surgery, varicocele, erectile dysfunction, testosterone deficiency, infertility, nutcracker syndrome, benign prostate hyperplasia (BPH), bladder cancer, prostate cancer, pelvic congestion, ovarian cancer, polycystic ovarian syndrome, endometriosis, and/or uterine fibroids.

Various embodiments of systems, methods, and devices are disclosed herein that make anastomosis easier and more time-efficient by enabling the use of anastomotic couplers in microsurgery and laparoscopy. Simplifying the anastomosis procedure minimizes required operator skill, reduces the duration of intense concentration, and helps reduce the surgeon's fatigue during long, complex operative procedures.

The present disclosure and the accompanying drawings are intended to describe some, but not necessarily all, examples or embodiments and does not limit the scope of the disclosure(s) in any way. Any referenced drawings herein may not be to scale and may be exaggerated in scale for convenience of explanation. In the explanation of several different examples of embodiments below, corresponding characteristics are provided with the same reference numbers.

For clarity of disclosure, the terms "proximal" and "distal" are defined herein relative to a human or robotic operator of the surgical instrument. The term "proximal" refers the position of an element closer to the human or robotic operator of the surgical instrument and further away from the surgical end effector of the surgical instrument. The term "distal" refers to the position of an element closer to the surgical end effector of the surgical instrument and further away from the human or robotic operator of the surgical instrument. In addition, the terms "upper," "lower," "lateral," "transverse," "bottom," "top," are relative terms to provide additional clarity to the figure descriptions provided below. The terms "upper," "lower," "lateral," "transverse," "bottom," "top," are thus not intended to unnecessarily limit the invention described herein.

Generally, the present disclosure relates to several systems, methods, and devices for applying anastomotic couplers in order to carry out minimally invasive vascular procedures, and in particular, performing anastomoses between hollow tissue structures when access to the tissue site is limited. In some embodiments, a device (occasionally referred to herein for the sake of brevity as anastomosis apparatus, apparatus, or surgical instrument) is configured for applying anastomotic couplers via a laparoscopic approach and comprises a proximal handle portion or interface, an intermediate shaft assembly of body extending distally from the handle portion, and an end effector arranged a distal end of the shaft assembly.

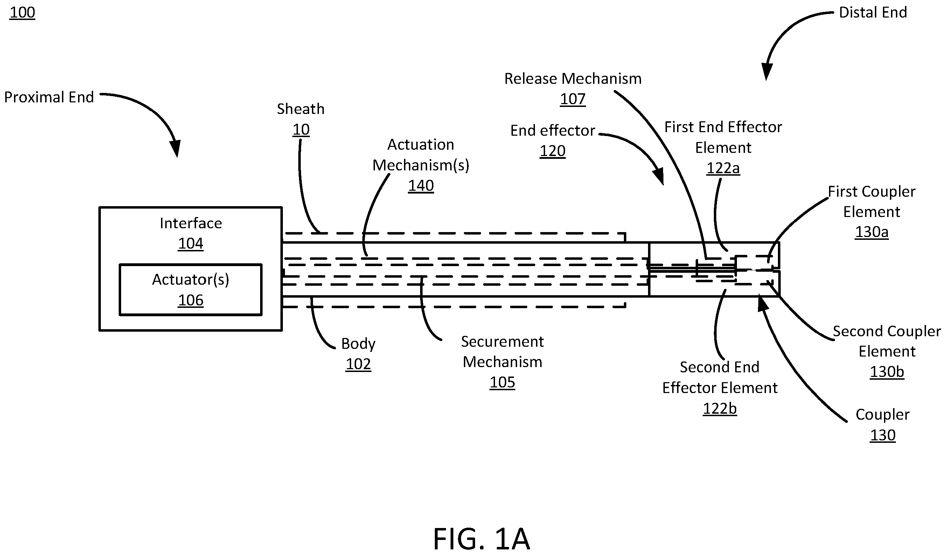

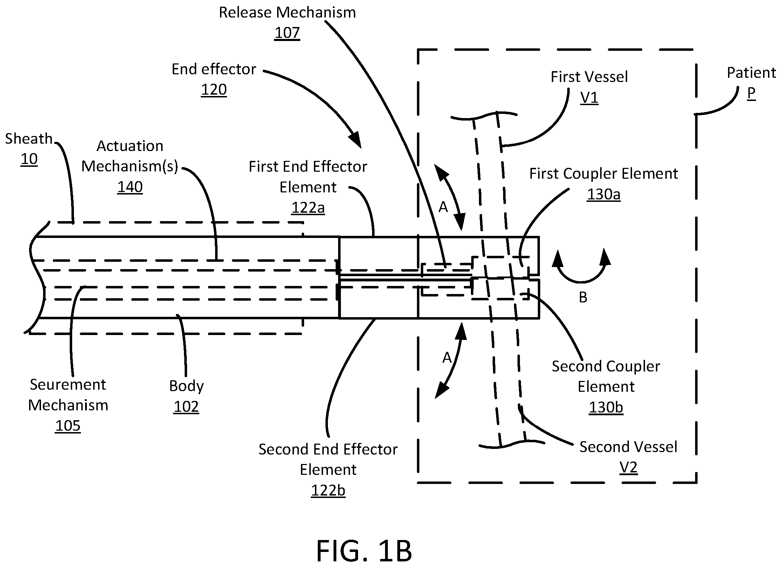

For example, FIG. 1A is a schematic illustration of an anastomosis apparatus 100 for performing laparoscopic anastomosis of a first vessel V1 to a second vessel V2, and a FIG. 1B is schematic illustration of a portion of the apparatus 100 of FIG. 1A illustrating the first vessel V1 and the second vessel V2 being coupled to each other via the apparatus 100 of FIG. 1A, according to an embodiment. The apparatus 100 includes a body 102 coupled to an interface 104 that may include one or more actuators 106 included therein or otherwise coupled thereto, an end effector 120 including a first end effector element 122a configured to removably receive a first coupler element 130a of a coupler 130 and a second end effector element 122b configured to removably receive a second coupler element 130b of the coupler 130. The apparatus 100 may optionally also include an actuation mechanism 140, a securement mechanism 105, and/or a release mechanism 107.

The body 102 may include an elongate member that may have a length in a range of 4 inches to 30 inches (10.16 to 76.2 cm), inclusive of all sub-ranges and values therebetween (e.g., 4, 6, 7, 8, 9, 10, 11, 12, 15, 18, 21, 25, 28 or 30 inches (10.16, 15.24, 17.78, 20.23, 22.86, 25.4, 27.94, 30.48, 38.1, 45.72, 53.34, 63.5, 71.12 or 76.2 cm), inclusive). In some embodiments, the body 102 may have a length of at least 8 inches (20.23 cm). The body 102 may be formed from any suitable material, for example, stainless steel, alloys, etc. The body 102 may define a longitudinal channel therethrough through which various components of the apparatus 100 may be disposed. A sheath 10 is be disposed around the body 102. The sheath 10 may be formed from any suitable material, for example, stainless steel, alloys, plastics, etc. A user may be able to selectively move the body 102 axially within the sheath 102, for example, to extend or withdraw the end effector at least partially into the sheath 10.

A proximal end of the body 102 is coupled to the interface 104 such that the elongate body extends longitudinally therefrom. The interface 104 may include a housing that may have an ergonomic shape for a user to grip. For example, in some embodiments, the interface 104 may be shaped as a handle or include a finger or hand grip facilitating the user in gripping the interface 104 and manipulating the interface 104 to perform laparoscopic anastomosis of two vessels or any other tubular structures.

One or more actuators 106 may be coupled to the interface 104. In some embodiments, the actuators 106 may include a push buttons, pull levers, triggers structured to be engaged by an index and/or middle finger of the user, thumb triggers configured to be engaged or otherwise manipulated by a thumb of the user, slide actuators, clips, levers, rotating knobs, scissor levers, or another first order level, any other suitable actuator, and/or combination thereof. In some embodiments, the apparatus 100 may also include an actuation mechanism 140 extending through one or more channels defined through the body 102 and operatively coupling the actuator(s) 106 to the end effector 120, for example, to cause a corresponding action or movement of the end effector 120. Such actuation mechanisms 140 may include, for example, rods, gears, rack and pinions, pistons, levers, bands, threads, ropes, pulleys, etc.

For example, the one or more actuators 106 may be configured to cause the first end effector element 122a and the second end effector element 122b to articulate about a distal end of the body 102 in one or more directions to separate from each other and/or to approach one each other. For example, the one or more actuators 106 can be configured to move the first end effector element 122a and the second end effector element 122b between a first configuration where the two end effector elements 122a, 122b are separated from one another and a second configuration where the two end effector elements 122a, 122b are adjacent to one another. In the second configuration, the two end effector elements 122a, 122b can be configured to couple the first coupler element 130a to the second coupler element 130b. In some embodiments, the first and second end effector elements 122a, 122b can also be configured to be in an intermediate configuration, e.g., between the first and second configurations. The one or more actuators 106 can be coupled to the actuation mechanism(s) 107, which can be driven by the one or more actuators 106 to cause movement of the first and second end effector elements 122a, 122b. In some embodiments, the actuator(s) 106 may be configured to cause one or more portions of the end effector 120 (e.g., the first end effector element 122a and/or the second end effector element 122b) to articulate in a single direction (e.g., a first direction), while in other embodiments, the actuator(s) 106 may be configured to cause one or more portions of the end effector 120 to articulate in multiple directions (e.g., a first direction and a second direction). For example, the first direction may involve rotation of a distal end of the first and second end effector elements 122a/b to move about the distal end of the body 102 towards or away from each other as indicated by the arrow A in FIG. 1B. Additionally or alternatively, the second direction may include articulation of the first and second end effectors 122a/b together in a direction that is substantially orthogonal to the first direction as indicated by the arrow B in FIG. 1B, thus allowing flexibility in positioning the end effector 120 at a desired location within a patient's P body. In some embodiments, a first actuator 106 can be configured to rotate the first end effector element 122a about a first axis, e.g., to move the first end effector element 122a toward or away from the second end effector element 122b. In some embodiments, a second actuator 106 can be configured to rotate the second end effector element 122b about a second axis, e.g., to move the second end effector element 122b toward or away from the first end effector element 122a. In some embodiments, the first axis about which the first end effector element 122a is configured to rotate and the second axis about which the second end effector element 122b is configured to rotate can be the same axis, while in other embodiments, the two axes can be offset from one another. In some embodiments, the first and second end effector elements 122a, 122b can also be pivoted about a third axis or angled away from a longitudinal axis of the body, e.g., to position the first and second end effector elements 122a, 122b for easier viewing by a user (e.g., a surgeon).

For example, in some embodiments, movement in the first direction may include articulation of the first and/or second end effector elements 122a/b in a plane extending along a longitudinal axis of the end effector 120, and the second direction may involve articulation of the first and second end effector elements 122a/b about the plane that extends along the longitudinal axis.

As shown in FIGS. 1A-1B, the end effector 120 is coupled to a distal end of the elongate body 102. The end effector 120 includes the first end effector element 122a defining a first receptacle structured to hold the first coupler element 130a of the coupler 130 and the second end effector element 122b defining a second receptacle structured to hold a second coupler element 130b of the coupler 130. The coupler 130 may include any suitable coupler that can be used to couple two vessels or any other tubular tissue structures. For example, the first coupler element 130a may include a ring-shaped member defining a central opening configured to receive an axial end of the first vessel V1. Similarly, the second coupler element 130b may also include a ring-shaped member configured to receive an axial end of a second vessel V2. In some embodiments, the second coupler element 130b may be a mirror image of the first coupler element 130a.

In some embodiments, each of the first and second coupler elements 130a/b may include coupling features, for example, mating snap-fit features (e.g., pins, grooves, slots, ledges, protrusions, notches, indents, detents, etc.) such that when corresponding surfaces of the first and second coupler elements 130a/b are pressed against each other, the first coupler element 130a is coupled to the second coupler element 130b. This also causes corresponding axial ends of the first vessel V1 and the second vessel V2 to be in contact with each other, which can lead to a coupling of the vessels V1 and V2 with each other. In some embodiments, axial ends of the first and the second vessel V1 and V2 may be flared out before coupling them together.

In some embodiments, the first end effector element 122a and the second end effector element 122b can be implemented as jaws or clamp arms that can articulate about their proximal ends such that the proximal ends of first and second end effector element 122a/b remain proximate to each other during operation, while the distal ends of the first and second end effector element 122a/b may be selectively moved away from each other to open or close the end effector 120. For example, the end effector 120 may be configured to move between a first configuration in which at least a portion of the first end effector element 122a (e.g., a distal end thereof) is separate from a corresponding portion of the second end effector element 122b (e.g., a corresponding distal end thereof) such that the first coupler element 130a is separate from the second coupler element 130b, and a second configuration in which at least the portion of first end effector element 122a is proximate to the corresponding portion of the second end effector element 122b such that the first coupler element 130a is coupled to the second coupler element 130b (e.g., as shown in FIG. 1B) so as to couple the first vessel V1 to the second vessel V2.

In some embodiments, the first and the second end effector elements 122a/b may be laterally spaced apart by a fixed or variable distance along their lengths, in the first configuration. The first end effector element 122a and/or the second end effector element 122b may then laterally displace toward each other until the first and second coupler elements 130a/b are coupled together in the second configuration. The lateral displacement can be a lateral translation.

In some embodiments, proximate ends of the first end effector element 122a and the second end effector element 122b may be coupled to a distal end of the elongate body 102. The first coupler element 130a and the second coupler element 130b may be removably coupled to corresponding distal ends of the first end effector element 122a and the second end effector element 122b, respectively. Moreover, the first and the second end effector elements 122a/b may be configured to articulate about the distal end of the elongate body 102 in the first direction (e.g., as indicated by the arrow A to move the end effector 120 between the first and the second configurations. In some embodiments, the proximate ends of the first and the second end effector elements 122a/b may be coupled to the distal end of the elongate body 102 via the actuation mechanism 140 that may be structured to allow articulation of the first and second effector elements 122a/b, as previously described.

In some embodiments, the actuation mechanism 140 may also be configured to move the first and the second end effector elements 122a/b into an intermediate configuration in which at least the portion of the first end effector element 122a (e.g., the distal end thereof) is located proximate to the corresponding portion of the second end effector element 122b but with a gap therebetween such that the first coupler element 130a is not coupled to the second coupler element 130b. This may advantageously reduce the lateral width of the end effector 120 to facilitate insertion into the body of the patient P through a small incision (e.g., having a diameter of less than 25 mm), thus reducing injury and allowing faster healing.

Any suitable actuation mechanism 140 may be used. In some embodiments, the actuation mechanism 140 may include a first linkage arm coupled to the first end effector element 122a and a second linkage arm coupled to the second end effector element 122b. Each of the first and a second linkage arms may include a central hinge such that moving a proximal end of each of the first and second linkage arms proximate to a corresponding distal end of the first and second linkage arm causes the first and second linkage arms to articulate about their respective central hinges away from each other so as to selectively move the end effector from the first configuration to the second configuration. In other words, the first and second linkage arms may serve as scissor arms to cause the first and second end effector elements 122a/b to move between the first and the second configurations.

In some embodiments, the actuation mechanism 140 may include at least one pulley coupled to a corresponding one of the first end effector element 122a and/or the second end effector element 122b. At least one tether may be coupled to the at least one pulley. The at least one tether may be configured to be displaced longitudinally (e.g., by a user via engagement of a corresponding actuator 106). Since the tether runs around the pulley, displacing the tether causes the at least one pulley to rotate and move the end effector between the first and the second configurations.

In some embodiments, the actuation mechanism 140 may include at least one rod coupled to a corresponding one of the first end effector element 122a or the second end effector element 122b and configured to rotate to cause the end effector 120 to move between the first and second configurations. For example, the at least one rod may be coupled to the corresponding one of the first end effector element 122a and/or the second end effector element 122b proximate to a radially outer edge of the corresponding one of the first end effector element 122a or the second end effector element 122b. Thus, rotating the rod causes the radially outer edges of the first and second end effector elements 122a/b that are located distal from the axial end of the rod to move proximate to or away from each other, thus moving the end effector 120 between the first and second configurations.

In some embodiments, the end effector 120 is withdrawn at least partially into a sheath 10 or the sheath 10 is axially displaced relative to the end effector 120 such that an inner surface of a distal end of the sheath 10 covers or surrounds an outer surface of each of the first and the second end effector elements 122a/b. In some embodiments, the sheath 10 may have a diameter that is less than or about equal to a maximum lateral width of the end effector 120 such that axial displacement of the sheath 10 toward the end effector 120 (or retraction of the end effector 120 within the sheath 10) urges the first and second end effector elements 122a/b into the second configuration.

In some embodiments, at least one closure element may be coupled to the first end effector element 122a and the second end effector element 122b and configured to urge the end effector towards the second configuration. The closure element may include, for example, mechanical linkages, rope wires, threads, sutures, filaments, extrusions, springs, rubber bands, bungee cords, any other suitable closure element, or any combination thereof. The closure element may be configured to bias the first end effector element 122a and the second end effector element 122b towards each other so as to facilitate moving the end effector 120 into the second configuration from the first configuration. In some embodiments, a resilient element may additionally, or alternatively, coupled to the first end effector element 122a and/or the second end effector element 122b, and configured to bias the end effector 120 towards the open configuration. The resilient element may include, for example, a biasing member such as a spring, a resilient plate (e.g., a NITNOL plate), mechanical linkages, rope wires, threads, sutures, filaments, extrusions, etc., and configured to bias the first and/or the second end effector element 122a/b towards the first configuration.

In some embodiments, each of first end effector element 122a and the second end effector element 122b may be configured to torsionally bend along their respective axis to move between the first configuration and the second configuration. For example, each of the first and second end effector elements 122a/b may include an actuating backbone that may be in the shape of a continuum structure with helical tendon wire routing. Such structures may include a set of disks through which a push and pull tendon wire is routed following a helical shape around the main axis of the structure. In other embodiments, the backbone may be reticulated. The actuation mechanism 140 may be configured to engage the tendon wire such that the backbone of each of the first and second end effector element 122a/b undergoes not only a pure rotation but also a torsion along the backbone main axis causing at least a distal end of the first and second end effector elements 122a/b to move away from each other to move the end effector 120 into the first configuration, or to move the first and second end effector elements 122a/b towards each other to move the end effector 120 int the second configuration. Such complex motion not only separates the distal tips of the first and second end effector elements 122a/b by bending the first and second end effector elements 122a/b away from each other, but may also expose the internal face and ultimately the first and second coupler elements 130a/b coupled thereto by rotating the first and second end effector about their respective longitudinal axis.

In some embodiments, each of the first end effector element 122a and the second end effector element 122b may include alignment features defined on corresponding surfaces thereof. The alignment features may be configured to align the first and the second end effector elements 122a/b as the end effector 120 moves into the second configuration, for example, to facilitate alignment of the first coupler element 130a with the second coupler element 130b. Such alignment features may include, but are not limited to, one or more notches, lips, grooves, indents, detents, protrusion, or other mating features that help with coarse and fine alignment of the first coupler element 130a with the second coupler element 130b during actuation of the apparatus 100 for moving the end effector 120 from the first to the second configuration. Moreover, the end effector 120 may include one or more grips, grooves, surface modifications, latching mechanisms, or any combination thereof to facilitate controlled anastomosis.

Optionally, in some embodiments, the apparatus 100 can include a securement mechanism 105 that may be configured to selectively secure the coupler 130 (e.g., the first coupler element 130a and/or the second coupler element 130b) to the end effector 122. Optionally, in some embodiments, the apparatus 100 can include a release mechanism 107, for example, to release the first coupler element 130a and/or the second coupler element 130b from the end effector 120. In some embodiments, the receptacles of the first and the second end effector elements 122a/b may include cutouts, cavities, slots, etc., shaped and sized to receive the first and second coupler elements 130a/b, respectively, snugly (e.g., via a friction fit). In some embodiments, the first and second coupler elements 130a/b may be selectively secured in their respective receptacles or released therefrom, e.g., via the securement mechanism 105 and/or the release mechanism 107. For example, the release mechanism 107 may be configured to be selectively actuated to release the coupler 130 from the end effector 120 in the second configuration. In some embodiments, the release mechanism 107 can be coupled to the securement mechanism 105, e.g., such that movement of the securement mechanism 105 can drive movement of the release mechanism 107 to release the coupler 130. Alternatively, the apparatus 100 may not include a securement mechanism 105 or a release mechanism 107. In such instances, a surgeon can use a separate tool (e.g., pliers, hooks, etc.) to disengage the coupler elements 130a/b from the end effector elements 122a/122b.

Any suitable securement mechanism 105 may be used. In some embodiments, at least one of the first coupler element 130a and the second coupler element 130b may define a circumferential groove on a radially outer surface thereof, for example, circumferentially around the first and/or second coupler element 130a/b in a radial edge thereof. In such embodiments, the securement mechanism 105 may include a string (e.g., a twine, a rope, a thread, a band, a chain, etc.) disposed in a portion of the circumferential groove to secure the first coupler element 130a and/or the second coupler element 130b in the first configuration within the end effector 120. The string may be configured to be selectively removed from the circumferential groove by a user (e.g., by engaging a corresponding actuator 106 to pull the string out of the circumferential groove) to release the first coupler element 130a and/or the second coupler element 130b in the first or the second configuration. For example, since the first and second coupler elements 130a/b may be joined to each other in the second configuration, releasing of one may cause release of both the coupler elements 130a/b.

**In** some embodiments, at least one of the first coupler element 122a and the second coupler element 122b defines an indent on a radially outer surface thereof (e.g., on a radially outer edge of the first coupler element 130a and/or the second coupler element 130b. In such embodiments, the securement mechanism 105 may include a rod having an axial end thereof disposed in the indent to secure the at least one of the first coupler element 130a and the second coupler element 130b in the first configuration (e.g., to prevent rotation of first coupler element 130a and/or the second coupler element 130b within the receptacle, or snap-fit in the indent to prevent axial displacement of the first and/or second coupler element 130a/b). The axial end of the rod may be configured to be selectively removed from the indent (e.g., caused by a user engaging an actuator 106 operatively coupled to the securement mechanism 105) to release the first coupler element 130a and/or the second coupler element 130b in the second configuration. In some embodiments, a biasing member (e.g., a spring such as a helical spring, a torsion spring, a Belleville spring, etc.) may be coupled to the rod and configured to bias the rod towards the at least one of the first coupler element 130a and the second coupler element 130b to secure them within their respective receptacles.

**In** some embodiments, the apparatus 100 may include the release mechanism 107 that is configured to selectively urge the first coupler element 130a and/or the second coupler element 130b out of the end effector 120. For example, the first and the second coupler elements 130a/b may be secured in their respective receptacles of the first end effector element 122a and the second end effector 122b via a friction fit such that a predetermined amount of force may be necessary to expel the first and the second coupler elements 130a/b from their respective receptacles.

**In** some embodiments, the release mechanism 107 may include a piston disposed in a portion of the first end effector element 122a or the second end effector element 122b. The piston may be configured to selectively protrude into the receptacle of the first end effector element 122a and/or the second end effector element 122b (e.g., in response to a user engaging a corresponding actuator 106) to urge (e.g., push) the first coupler element 130a and/or the second coupler element 130b out of the end effector 120. For example, once the first coupler element 130a and the second coupler elements 130b are coupled to each other in the second configuration of the end effector 120 to couple the first vessel V1 to the second vessel V2, as previously described, a user may engage the corresponding actuator 106 to push the piston into the receptacle. The piston is axially extended into the receptacle(s) until the first and second coupler elements 130a/b are released from their respective receptacles. The first and second coupler elements 130a/b can be left in the patient P's body while the elongate body 102 and thereby, the end effector 120 are withdrawn from the patient P's body.

The first vessel V1 and the second vessel V2 may be located in any part of a patient's body such that the apparatus 100 may be equally applicable in performing various laparoscopic anastomosis procedures including, but not limited to, varicocele repair, cardiovascular surgery, erectile dysfunction, testosterone deficiency, infertility, nutcracker syndrome, BPH, bladder cancer, prostate cancer, pelvic congestion, ovarian cancer, polycystic ovarian syndrome, endometriosis, and/or uterine fibroids. Thus, the apparatus 100 or any other anastomosis apparatus described herein make anastomosis easier and more time-efficient by enabling the use of anastomotic couplers in microsurgery and laparoscopy. Simplifying the anastomosis procedure minimizes required exercise of operator skill, reduces the duration of intense concentration, and helps reduce the surgeon's fatigue during long, complex operative procedures.

The apparatus 100 or any other anastomosis procedures described herein may be particularly beneficial in treating the following conditions:

**Varicocele Repair:** Varicocele is a chronic condition in which the testicular veins become enlarged from the pooling of venous blood, causing venous reflux and significantly reducing testicular blood flow. The method to treat varicocele is called varicocelectomy. Since 1929, the most popular method of varicocelectomy has been focused on ligation of testicular veins and preventing reflux only.

The left internal spermatic vein (ISV) merges with the left renal vein at a right angle near the superior mesenteric artery, while the right internal spermatic vein tends to merge with the inferior vena cava at more of an acute angle. One-way valves within the internal spermatic vein (ISV) prevent back-flow of venous blood draining from the testicles. Venous blood from the testicles must flow upwards against gravitational forces that are the highest while standing. The left internal spermatic vein (ISV) merges with the left renal vein at a right angle near the superior mesenteric artery, while the right internal spermatic vein tends to merge with the inferior vena cava at more of an acute angle. The repetitive loading of the one-way valves in the ISVs (a consequence of the bipedal, or upright, posture in humans) frequently leads to gradual deterioration and eventual failure of the valves; a consequence of the bipedal, or upright, posture in humans.

This chronic reduced blood flow can result in a pathophysiological condition called varicocele. Varicocele is associated with the left ISV in 95% of the cases. This is a result of a variety of anatomical differences, particularly vessel length. Effectively, the left ISV is more readily diagnosed and has been widely linked to male infertility. Varicocele can also be caused by the compression of the renal vein by the superior mesenteric artery and the aorta. This results in the nutcracker phenomenon (NCP) or left renal vein entrapment. The prevalence of varicocele has been shown to increase with age, reaching over 75% at the age of 70. Several studies have demonstrated a strong correlation between varicocele and left renal vein compression as well as the deterioration of one-way valves that leads to bilateral vascular disease.

Currently, varicocele is treated by ligation through multiple different access points. The ISV is ligated through an open surgical procedure (microsurgical/surgical ligation through inguinal, sub-inguinal access), laparoscopic procedure through abdominal access points or interventional/percutaneous procedure through femoral or carotid vein access points. In a typical laparoscopic varicocele ligation procedure, a camera and several small instruments are introduced to the abdomen. The peritoneum is dissected to get access to the spermatic vein. The ISV is carefully separated from the artery and lymphatics. The ISV is ligated and clipped, and the tools are retracted. In contrast, the apparatus 100 may be used to perform varicocele repair via a minimally invasive procedure in less than 5 minutes, that allows for shorter hospital stays and faster healing times.

There are several advantages to using a fluid connection to treat varicocele, including, for example: typically a single connection can be made for an entire testicular vein bed to equalize testicular venous blood pressure, including its collaterals; the approach can be used in patients with nutcracker syndrome or left renal vein compression, as it allows for an alternative pathway for renal venous blood drainage apart from renal veins; avoids the need for multiple embolization coils to ensure complete blockage of the spermatic vein; etc. The use of a fluid connection also reduces the exposure of the prostate to free testosterone compared to embolization or ligation of the testicular vein(s). The testicular veins are the primary route for drainage of testicular venous blood. Ligation or occlusion of the testicular vein(s) forces blood drainage through other smaller blood vessels into the iliac vein. Since the back pressure of the testicular venous blood following ligation or occlusion of the testicular vein(s) is reduced, the exposure of the prostate to testosterone-rich venous blood is reduced for a patient receiving this treatment. However, with ligation or occlusion, the prostate would still be exposed to higher-than-normal, supra-physiological levels of testosterone concentrations. Conversely, in accordance with embodiments described herein, creating a fluid connection using the apparatus 100 or any other apparatus described herein, can allow for drainage of the majority of testosterone-rich blood directly into the greater venous blood vessels, thereby reducing exposure of the prostate to testosterone-rich blood. The use of a fluid connection can also lead to immediate, long-standing pain relief, while inflammation of the spermatic cord and testicular vein(s) can reoccur post-occlusion or post-ligation as the testicular vein, and its collaterals, further degrade over time.

**Tissue Transplantation:** The transplantation of tissue from one part of a patient's body to another provide a means for reconstructive surgeons to repair and replace body parts, restoring appearance and in many cases function and feeling. The most common reasons for patients to undergo tissue transfers is after tumor extirpation (e.g., breast cancer reconstruction), trauma, burn injury, or to restore absent function associated with congenital anomalies. In these tissue transfers, the microsurgeon removes tissue, including skin, fat, muscle, nerves and bone, with an associated vascular pedicle, from one part of the body and moves it to the part of the body where it is needed for aesthetic or functional restoration. The arteries and veins are re-attached, and in some cases, the nerves are as well. The apparatus 100 may be easily applied to tissue transplantation procedures to reconnect arteries, veins, and/or nerves in a fast, and minimally invasive manner.

**Cardiovascular Surgery:** One of the most common surgical procedures carried out today which requires performing an anastomosis is coronary artery bypass grafting (CABG), commonly referred to as bypass surgery. This procedure is used to treat patients suffering from coronary disease in the form of one or more coronary arteries that are partially or completely blocked by stenoses. When blood flow through the coronary arteries is restricted or occluded, the cardiac muscle tissue becomes deprived of adequate blood flow, which eventually results in death of the muscle tissue. Interventional procedures other than bypass surgery, for example, angioplasty and atherectomy, are also used to treat occluded coronary arteries. However, bypass surgery is usually desirable or necessary to treat patients suffering from severe or multiple coronary artery blockages, or when other interventional procedures have been or would likely be unsuccessful.

In order to bypass a blockage in a coronary artery, the surgeon must anastomose a vascular conduit which is in communication with a source of arterial blood to the coronary artery at a location downstream of the blockage. The vascular conduit may be a native artery carrying blood from the patient's heart, for example, the right or left internal mammary artery (IMA). In such cases, the artery may be transected from the patient's body to provide a free end which is prepared for distal anastomosis to the coronary artery. Alternatively, the IMA may be transected and removed from the body and one end prepared for anastomosis to an arterial blood source and the other to a coronary artery. Further, depending on the number of coronary arteries which are blocked, in addition to using the right and/or left IMA, other vascular conduits may be needed. One end of each conduit is prepared for distal anastomosis to the coronary artery, while the other end is prepared for proximal anastomosis to an arterial blood source, for example, the aorta. The vascular conduits may be harvested from the patient's body, suitable examples of which include the left or right IMA, inferior epigastric artery, splenic artery, subclavian artery, saphenous vein, etc. Also, animal or synthetic vascular conduits may be used instead of or in addition to those mentioned above.

The most common form of bypass surgery involves bypassing blockages in multiple coronary arteries, e.g., quadruple, five or six-way bypass procedures. As a result, most bypass procedures require a number of vascular conduits to form the necessary anastomoses. However, there is a limited number of native arterial conduits available which may be used by simply attaching one end to a blocked coronary artery. As such, it is usually necessary to use free conduits or grafts, which requires forming an anastomosis at both ends of each conduit, one end to an arterial blood source and the other end to the blocked coronary artery. The patient's aorta is a desirable arterial blood source to which the proximal end of one or more conduits may be anastomosed. As is the case with all other anastomoses, the surgeon must securely suture the proximal end of each conduit to the patient's aorta in order to obtain a strong, fluid tight connection, which is a highly technical and time consuming procedure. Nevertheless, when performing bypass surgery via conventional, open-chest procedures in which the patient's sternum is split and retracted, the surgeon has essentially unobstructed access to the heart and aorta, which reduces the difficulty of forming the proximal anastomoses between the vascular conduits and the patient's aorta.

During the last several years, however, there has been a movement away from open-chest surgery toward minimally invasive cardiac surgery. These procedures are typically carried out through incisions made between the ribs, which requires surgeons to operate with considerably less access to the heart and aorta as compared to open-chest procedures. This reduced access to the heart has increased the difficulty and time associated with performing a vascular anastomosis. The already highly technical procedure is even more difficult when the surgeon is operating through a small incision or trocar port. The devices and methods used in conventional open-chest cardiac surgery are not always usable or readily adaptable to carry out minimally invasive cardiac surgery. Although anastomotic couplers are frequently employed in open procedures to join various tissue structures, they are not designed or easily adaptable for use in minimally invasive cardiac surgery. In contrast, the apparatus 100 or any of the anastomosis apparatus described herein may be used to easily access the heart via a small incision and rapidly perform vascular anastomosis.

While the present disclosure describes some clinical conditions that may benefit from laparoscopic procedures performed by the apparatus described herein, the apparatus 100 or any of the apparatus described herein may be used for any clinical or surgical procedure. Examples or such clinical conditions, particularly Varicocele, are described in PCT Application No. US2020/062287, filed 11/25/2020, and entitled "Systems, Apparatus, and Methods for Treatment of Varicocele and Associated Conditions,".

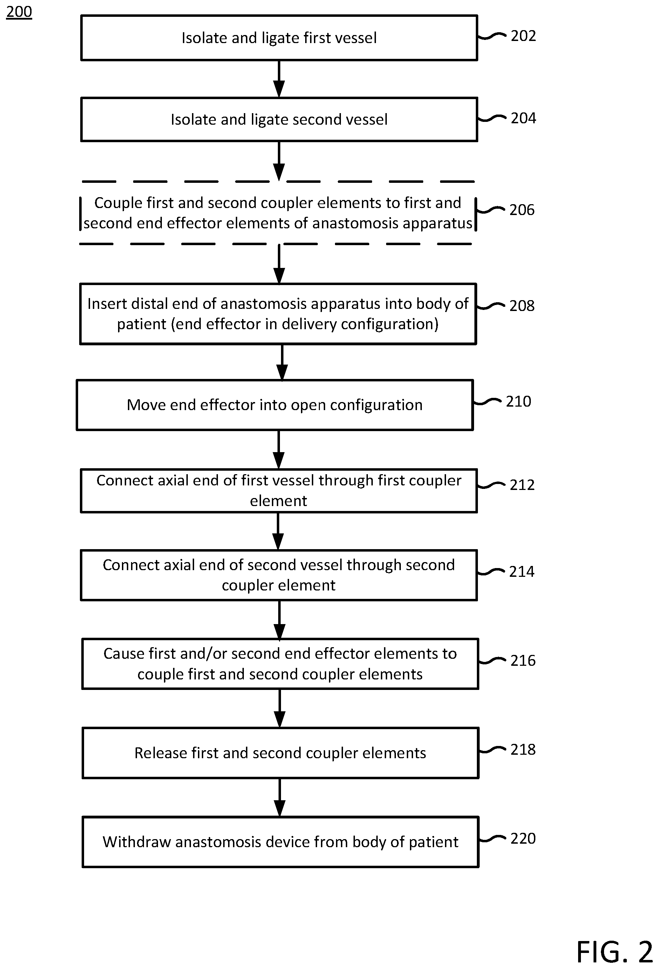

FIG. 2 is a schematic flow chart of a method 200 (which does not fall within the scope of the claims) for laparoscopically coupling a first vessel (e.g., the first vessel V1) to a second vessel (e.g., the second vessel V2) using an anastomosis apparatus (e.g., the anastomosis apparatus 100), according to an embodiment. While described with respect to the apparatus 100, the operations of the method 200 may be implemented using any of the anastomosis apparatus described herein. All such embodiments are contemplated and should be considered to be within the scope of the present disclosure.

The method 200 includes ligating the first vessel V1, at 202, for example, using via a clip, a band, a clamp, etc. The method 200 also includes ligating the second vessel V2, at 204. In some embodiments, the method 200 may include coupling the first coupler element 130a to the receptacle of the first end effector element 122a and coupling the second coupler element 130b to the receptacle of the second end effector element 122b, at 206. For example, the apparatus 100 may be provided to a user with the coupler 130 being separate from the end effector 120. In other embodiments, the apparatus 100 may be provided with the first and second coupler elements 130a/b already coupled to the first and second end effector elements 122a/b, respectively, thus obviating operation 206.

At 208, a distal end of the apparatus 100 is inserted into the body of the patient P. For example, a small incision having a diameter of equal to or less than 25 mm may be made in the body of the patient P and a distal end of the elongate body 102 and thus, the end effector 120 may be inserted into the body of the patient P. In some embodiments, the end effector 120 may be moved into the intermediate configuration as previously described before inserting the end effector 120 into the body of the patient P so as to facilitate insertion.

At 210, the end effector 120 is moved into the open configuration (i.e., the first configuration). For example, after inserting the end effector 120 into the body of the patient P, the user may engage the actuator 106 (e.g., a corresponding one of a set of actuators 106) to cause the first and second end effector elements 122a/b to articulate about a distal end of the elongate body away from each other.

At 212, an axial end of the first vessel V1 is connected through the first coupler element 130a. At 214, and axial end of the second vessel V2 is connected through the second coupler element. For example, the axial end of the first vessel V1 may be inserted through the throughhole defined through the first coupler element 130a and the axial end of the second vessel V2 inserted through the throughhole defined through the second coupler element 130b so as to connect the first and second vessels V1, V2 to the first and second coupler elements 130a/b, respectively. In some embodiments, the axial ends of the first vessel V1 and the second vessel V2 may be flared after insertion through the first and second coupler elements 130a/b, respectively. In some embodiments, pins, protrusions, notches, grooves, or any other securement elements may be provided on respective surfaces of the first and second coupler elements 130a/b that face each other. The respective axial ends of the first and second vessel V1 and V2 may be connected to the first and second coupler elements 130a/b, respectively by coupling the axial ends to the securement elements.

At 216, the first end effector element 122a and/or the second end effector element 122b are caused to move into a closed configuration (i.e., the second configuration) to couple the first coupler element 130a to the second coupler element 130b, thus coupling the first vessel V1 to the second vessel V2, as previously described. At 218, the first and second coupler elements 130a/b are released from the first and second end effector elements 122a/b, for example, via releasing the first and/or second coupler element 130a/b from the securement mechanism 105 and/or activating the release mechanism 107, as previously described. Alternatively, a surgeon can use a separate tool to pull or otherwise disengage the first and second coupler element 130a/b from the first and second end effector elements 122a/b. At 220, the apparatus 100 is withdrawn from the body of the patient P, for example, by withdrawing the elongate body 102 and thus, the end effector 120 form the body of the patient P. The end effector 120 may be maintained in the closed configuration during withdrawal from the patient P's body. The laparoscopic incision is closed (e.g., sutured, stapled, or glued) to end the surgical procedure.

Referring to FIGS. 3-4C, FIG. 3 is a side perspective view of an anastomosis apparatus 300 with an end effector 320 of the apparatus 300 in a first (open) configuration, and an enlarged view of the end effector 320, according to an embodiment. The apparatus 300 may include a handle assembly 301 (e.g., an interface), a shaft assembly 302 extending longitudinally and distally from handle assembly 301, and an end effector 320 arranged at a distal end of shaft assembly 302. The handle assembly 301 may include a main body 303 including a pistol grip 304 and an anastomotic control button 306a (e.g., a first actuator) configured to be manipulated by a user to control various aspects of the application of anastomotic coupler rings 330a and 330b (e.g., first and second coupler elements) to target blood vessels (e.g., the vessels V1 and V2) as well as their release from the end effector 320 of the surgical instrument 300. A trigger 306 (e.g., a second actuator) may be coupled to a lower portion of main body 303 and is pivotable toward and away from the pistol grip 304 to selectively actuate the end effector 320. In some embodiments the handle assembly 301 may include a scissor grip configuration. Main body 303 may also be referred to herein as a housing 303 and may include one component or an assembly of components. The terms "body" and "housing" are thus not intended to unnecessarily limit the embodiments described herein to any number of discrete components.

As shown FIG. 3, the end effector 320 includes a first clamping arm 322a and a second clamping arm 322b (e.g., first and second end effector elements) configured to selectively pivot toward or away from each other for the purposes of applying or joining together the ends of two tubular structures such as arteries, veins, and/or nerves. Each clamp arm 322a/b may be operatively coupled with trigger 306b such that the set of clamp arms 322a/b are configured to pivot towards each other in a closed position, for example, in response to pivoting of the trigger 306b toward pistol grip 304. Further, the set of clamp arms 322a/b may be configured to pivot away from a closed position to an open position in response to pivoting of trigger 306b away from pistol grip 304. Various suitable ways in which clamp arms 322a/b may be coupled with trigger 306b will be apparent to those of ordinary skill in the art in view of the present disclosure. In some versions, one or more resilient elements may be incorporated to bias clamp arms 322a/b and/or trigger 306b towards the open position.

Shaft assembly 302 of the apparatus 300 extends along a longitudinal axis and may include an outer tube 309, an inner tube 310 dispose through outer tube 309, and a coupler release rod (not shown) supported within and extending longitudinally through the inner tube 310. A proximal end of each clamp arm 322a/b may be pivotally coupled to distal ends of outer tube 309 and/or and inner tube 310, enabling clamp arms 322a/b to pivot relative to shaft assembly 302 about a pivot axis defined by a pivot pin 311 extending transversely through the distal end of inner tube 310. In some embodiments, the shaft assembly 302 may alternatively include one or more lumens extending through shaft assembly 302 to the end effector 320, which may be configured for actuated control of surgical instrument 300 for performing anastomosis, including one or more pusher rods or actuation cables.

In some embodiments, as shown in FIG. 3, the inner tube 310 may be longitudinally fixed relative to handle assembly 301, and the outer tube 309 may be configured to translate relative to inner tube 310 and handle assembly 301, along the longitudinal axis of shaft assembly 302. As outer tube 309 translates distally, each clamp arm 322a/b may pivot about its pivot axis toward its open position. As the outer tube 309 translates proximally, each clamp arm may pivot about its pivot axis in an opposite direction toward its closed position. Though not shown, in some embodiments, a proximal end of the outer tube 309 may be operatively coupled with trigger 306b such that actuation of the trigger 306b may cause translation of outer tube 309 relative to inner tube 310, thereby opening or closing of the set of clamp arms 322a/b, as previously. In some embodiments, the outer tube 309 may be longitudinally fixed and the inner tube 310 may be configured to translate for moving the set of clamp arms 322a/b between the open and closed positions. Various other suitable mechanisms for actuating the set of clamp arms 322a/b may be used and should be considered to be within the scope of the present disclosure.

In some embodiments, the shaft assembly 302 and end effector 320 may be configured to rotate together relative to body 304 out the longitudinal axis defined by shaft assembly 302. As shown in FIG. 3, shaft assembly 302 may include a rotation knob 312 arranged at a proximal end thereof as well as a shaft coupler configured to mechanically connect to body coupler of handle assembly 301. Rotation knob 312 may be rotatably coupled to main body 304 of handle assembly 301 and may be rotationally fixed to outer tube 309 and inner tube 310 (e.g., by a coupling pin extending transversely therethrough). In other examples, the rotation knob 312 may be rotationally fixed to the remaining components of shaft assembly 302 in any suitable manner. The rotation knob 312 may be configured to be gripped by an operator to selectively manipulate the rotational orientation of shaft assembly 302 and end effector 303 relative to handle assembly 301.

Referring to FIGS. 4A-4C, FIG. 4A is a top perspective view of the end effector 320 of FIG. 3 in the first configuration within a body of a patient with a first vessel V1 inserted through a first coupler ring 330a coupled to the first clamp arm 322a and a second vessel V2 inserted through a second coupler ring 330b coupled to the second clamp arm 322b. The pair of anastomotic coupling rings 330a/b define throughholes 332a/b through the respective first vessel V1 and the second vessel V are inserted. The first coupler ring 330a is disposed in a first receptacle 324a of the first clamp arm 322a and the second coupler ring 330b is disposed in a second receptacle 324b defined in the second clamp arm 322b. The coupler rings 330a/b are configured to couple the tubular tissue structures V1, V2 to the clamp arms 322a/b.

The coupler rings 330a/b may include a plurality of fasteners operable to puncture the tubular structure and be joined together to form an anastomosis. In some embodiments, the coupler rings 330a/b can be removably coupled with the clamp arms 322a/b, for example, after coupling the first vessel V1 to the second vessel V2 as shown in FIG. 4C. Accordingly, the coupler rings 330a/b may be replaceable to allow multiple uses of the surgical instrument 300. In some embodiments, the coupler rings 330a/b may not be removable such that the surgical instrument 300 is provided for a one-time use. The surgical instrument 300 may include a pusher rod operably coupled to anastomotic control button 306a to controllably release the coupler rings 330a/b from the clamp arms 322a/b.

FIG. 4B is a top perspective view of the end effector 320 in the second configuration in which the clamp arms 322a/b close so as to couple the first coupler ring 330a to the second coupler ring 330b to couple the first vessel V1 to the second vessel V2. FIG. 4C shows the apparatus 300 being removed from the coupler rings 330a/b leaving the coupler rings 330a/b within the body of the patient with the first vessel V1 being coupled to the second vessel V2.

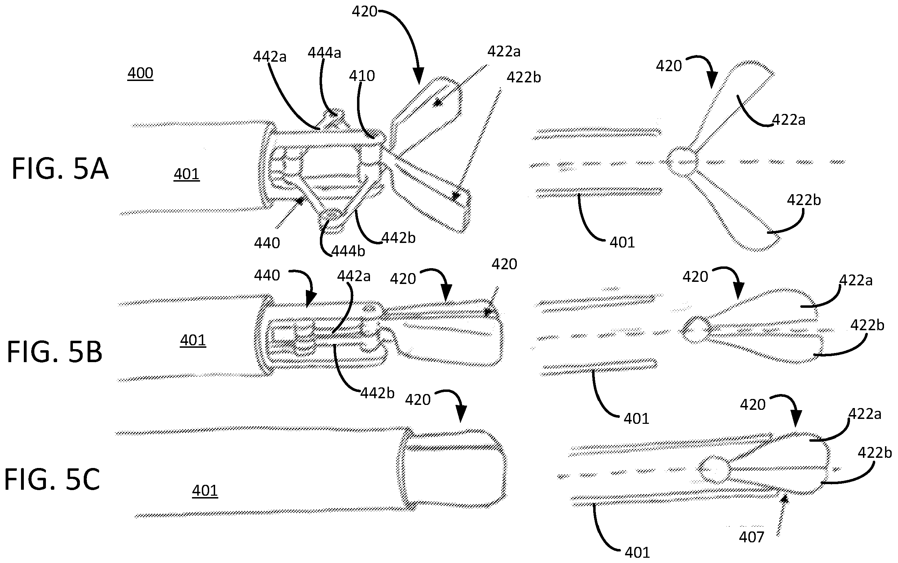

FIG. 5A shows a side perspective view and a side view of a portion of an anastomosis apparatus 400, according to an embodiment. FIG. 5B is a side perspective view and a side view of the portion of the apparatus 400 of FIG. 5A in an intermediate configuration, and FIG. 5C is a side perspective view and a side view of the portion of the apparatus 400 of FIG. 5A in a second configuration. The apparatus 400 includes a sheath 401, an actuation mechanism 440 including a first and second linkage arms 442a and 442b, and an effector 420 including clamp arms 442a and 442b (e.g., first and second end effector elements). The clamp arms 442a/b may be configured to pivot about the actuation mechanism 440 that may include a non-conventional revolute joint formed by the first and second linkage arms 442a/b. For example, the actuation mechanism 440 may include the first linkage arm 442a coupled to the first clamp arm 422a and the second linkage arm 442b coupled to the second clamp arm 422b. Each of the first and second linkage arms 442a/b may include a central hinge 444a/b. Moreover, proximate ends of the clamp arms 422a/b may be coupled to a distal end of the actuation mechanism 440 via a pivot joint 410 such that moving a distal end of each of the first and second linkage arms 442a towards a corresponding proximate end of the first and second linkage arms 422a, causes the first and second linkage arms 442a/b to articulate about their respective central hinge 444a/b away from each other so as to selectively move the end effector 420 from the open configuration to the closed configuration. In some embodiments, the end effector 420 may at least be partially withdrawn into the sheath 401 as shown in FIG. 4C to move the end effector 420 into the closed configuration.

Traditionally, laparoscopic jaws pivot about a rotating joint that is perpendicular to the main axis of the apparatus or device. This results in a planar motion of the end effector. In contrast, the apparatus 400 allows better visibility, exposure, access to the inner face of the clamp arms 422a/b via the angled and pivoting linkage arms 442a/b. By determining the desired configuration of the clamp arms 422a/b in the closed and open configurations, it may be possible to determine the optimal axis of rotation, hence joint angles with respect to the apparatus 400 main axis, such that the clamp arms 422a/b may achieve a three-dimensional (non-planar) trajectory in moving between the open and closed configurations.

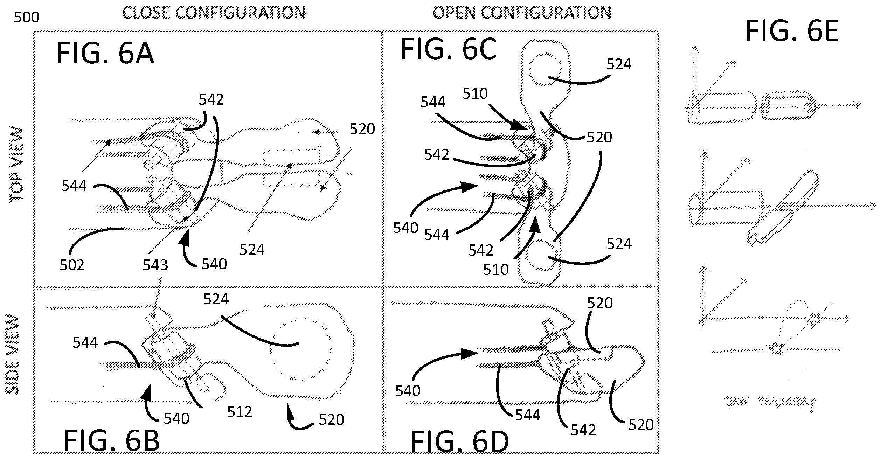

FIG. 6A is a top view, and FIG. 6B is a side view of a portion of an anastomosis apparatus 500 in a second (closed) configuration, according to an embodiment. FIG. 6C is a top view and FIG. 6D is a side view of the portion of the apparatus 500 in a first (open) configuration. FIG. 6E is a schematic illustration of a trajectory of an end effector of the apparatus of FIGS. 6A-6D between the first and the second configurations. The apparatus 500 includes an end effector 520 including clamps arms, defining receptacles 524, that are coupled at proximate ends thereof to an elongate body 502 via an actuation mechanism 540. The actuation mechanism 540 may include pulleys 542 coupled to a corresponding one of the first and second clamp arms 520. At least one tether 544 is coupled to the corresponding pulley 542 and configured to be displaced longitudinally to cause one or both pulleys 542 to rotate and move the end effector 520 between the first and the second configurations.

In some embodiment, any of the anastomosis apparatus described herein may have a dual actuation mode, for example: 1) a first actuation mode that allows large motion of the end effector; and 2) a second actuation mode that include fine and rigid closure of clamp arms (e.g., in a direction indicated by the arrows A in FIG. 1B). The first actuation mode may enable for the apparatus to open the end effector clamp arms quickly (e.g., at a rate of greater than about 180 deg/sec) and easily (e.g., with one degree of freedom) to perform its desired task. This can be achieved via any mechanism (e.g., mechanical linkages, nitinol ribbon, wire, ropes, etc.). The latter actuation mode may enable slow and controlled generation of high clamping forces at the tips of the clamp arms. This may be achieved by translating the outermost shaft of the apparatus over the jaw that is designed with a dedicated interference angle.

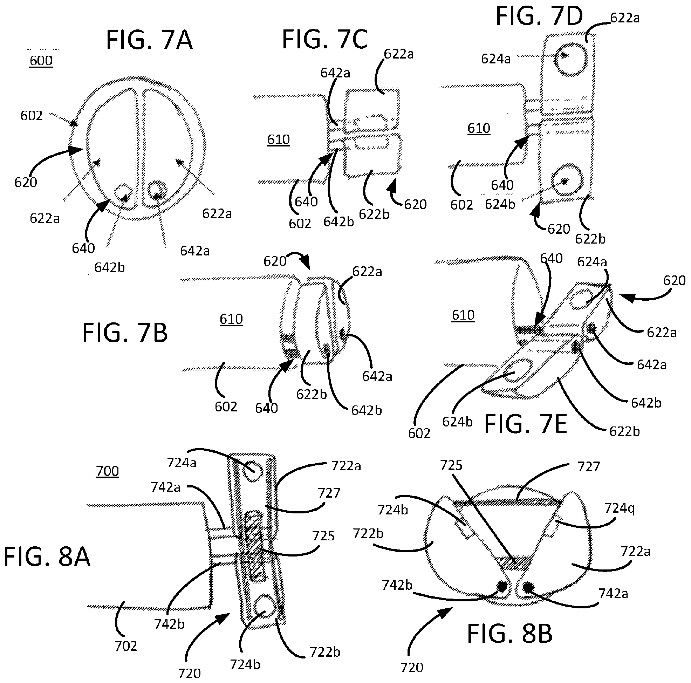

FIG. 7A is a front view, FIG. 7B is a side perspective view, and FIG. 7C is a top view of a portion of an anastomosis apparatus or surgical instrument 600 in a second (closed) configuration, according to an embodiment. FIG. 7D is a top view and FIG. 7E is a side perspective view of the portion of the apparatus 600 in a first (open) configuration. The apparatus 600 may include a shaft assembly 602 includes an end effector 620 including clamps arms 622a/b defining receptacles 624a/b. The apparatus 600 includes an actuation mechanism 640 that include a first rod 642a coupled to the first clamp arm 622a and a second rod 642b coupled to the second clamp arm 622b and configured to rotate to cause the end effector 620 to move between the first and second configurations. For example, the rods 642a/b may be coupled to the clamp arms 622a/b, respectively, proximate to a radially outer edge of thereof, as shown in FIGS. 7A-7E.

Expanding further, the apparatus 600 may include a substantially similar handle assembly and shaft assembly to surgical instrument 100, 300, including the shaft assembly 602. As shown in FIGS. 7A-7E, end effector 620 includes the set of pivotable clamp arms 622a/b configured to selectively pivot toward or away from each other for the purposes of applying or joining together the ends of two tubular structures such as arteries and/or veins. Each clamp arm 622a/b may be operatively coupled to an actuator (e.g., the trigger 306b) such that the set of clamp arms 622a/b are configured to rotate towards a closed position, in response to pivoting of actuator (e.g., moving the trigger 306b toward pistol grip 304). Further, the set of clamp arms 322a/b may be configured to rotate away from a closed position to the open position in response to opposite motion of the actuator (e.g., pivoting of trigger 306b away from pistol grip 304).

Shaft assembly 602 extends along a longitudinal axis and may include an outer tube (not shown), and an inner tube 610 received within outer tube, and may optionally, include a coupler release rod (not shown) supported within and extending longitudinally through inner tube 610. A proximal end of each clamp arm 622a/b may be rotationally coupled to distal ends of outer tube and inner tube 610 via the rods 642a/b enabling each clamp arm 622a/b to rotate relative to shaft assembly 602 about a pivot axis defined by rods 642a/b (e.g., pivot pins) extending transversely through the distal end of inner tube 610.

**In** some embodiments, the inner tube 610 may be longitudinally fixed relative to handle assembly (e.g., handle assembly 301). As actuator (e.g., trigger 306b) is pulled, each clamp arm 622a/b rotates about the pivot axis defined by rods 642a/b toward its closed position. As actuator (e.g., trigger 306b) is released, each clamp arm 622a/b rotates about the pivot axis in an opposite direction toward its open position. Various other suitable mechanisms for actuating the set of clamp arms 308 between the open and closed positions may be used and should be considered to be included within the scope of this disclosure.

**In** some embodiments, the shaft assembly 602 and the end effector 620 may be configured to rotate together relative to the interface (e.g., body 301) out of the longitudinal axis defined by shaft assembly 602. The surgical instrument 600 may also include a pair of anastomotic coupler rings (e.g., the coupler rings 330a/b) disposed in the receptacles 624a/b and configured to couple the tubular tissue structures (e.g., vessels V1 and V2) to the clamp arms 622a/b. The coupling rings may include a plurality of fasteners operable to puncture the tubular structure and be joined together to form an anastomosis. In some embodiments, the coupler rings can be removably coupled with the clamp arms 622a/b. Accordingly, the coupler rings may be replaceable to allow multiple uses of the surgical instrument 600. In some embodiments, the coupler rings may not be removable such that the surgical instrument 600 may be provided for a one-time use. The surgical instrument 600 may include a pusher rod operably coupled to a corresponding actuator (e.g., the anastomotic control button 306a) to controllably release the coupler rings (e.g., the coupler rings 330a/b) from the clamp arms 622a/b.

In some embodiments, the end effector 600 or any of the end effectors described herein may additionally include one or more closure elements to assist with complete closure of the coupler rings. In some embodiments, one or more resilient members may be incorporated to bias clamp arm and/or actuators toward the open position. For example, FIG. 8A is a top view and FIG. 8B is a front view of an apparatus 700 that includes resilient element 725 and closure elements 727, according to an embodiment. The apparatus 700 includes a shaft assembly 702, an end effector 720 including clamp arms 722a/b defining receptacles 724a/b for receiving coupler rings, and pivot rods 742a/b coupled to the respective clamp arms 722a/b, as described in detail with respect to the apparatus 600. The resilient element 725 may be coupled to each of the clamp arms 722a/b and may include a biasing member such as a spring, a resilient plate (e.g., a nitinol plate), mechanical linkages, rope wires, threads, sutures, filaments, extrusions, etc., and configured to bias the clamp arms 722a/b towards the open configuration. Moreover, the closure element 727 may be coupled to each clamp arm 722a/b. The closure element(s) 727 may include, for example, mechanical linkages, rope wires, threads, sutures, filaments, extrusions, springs, rubber bands, bungee cords, any other suitable closure element, or any combination thereof, and may be configured to bias the clamp arms 722a/b towards the closed configuration.

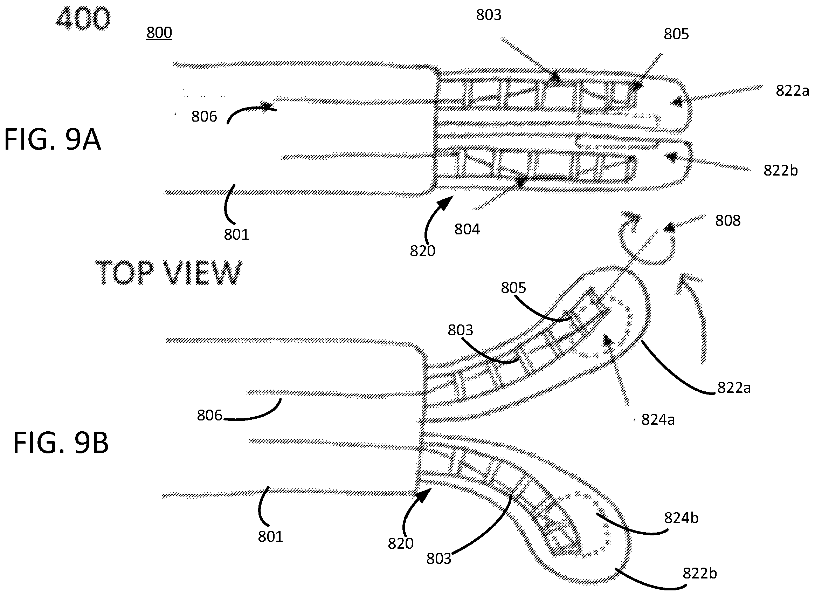

FIG. 9A is top view of a portion of an anastomosis apparatus 800 that includes an end effector 820 including a first clamp arm 822a and a second clamp arm 822b that are configured to bend along a longitudinal axis thereof, in a second (closed) configuration, and FIG. 9B is a top view of the apparatus 800 in which the end effector 820 is in a first (open) configuration, according to an embodiment. The clamp arms 822a/b define receptacles 824a/b configured to removably receive coupler rings (e.g., coupler rings 330a/b). In some embodiments, the clamp arms 822a/b may include with an actuating backbone 803, 804 that may be in the shape of a continuum structure with helical tendon routing. Such structures may include a set of disks 805 through which a push and pull tendon wire 806 may be routed following a helical shape around the main axis of the structure. By pulling the tendon wire, the backbones 803 and/or 804 may experience not only a pure rotation but also a torsion along the backbone main axis 408. Such complex motion may not only separate the distal tips of the clamp arms 822a/b but may also expose their internal face and ultimately the coupler rings that may be embedded to otherwise disposed within the receptacles 824a/b.

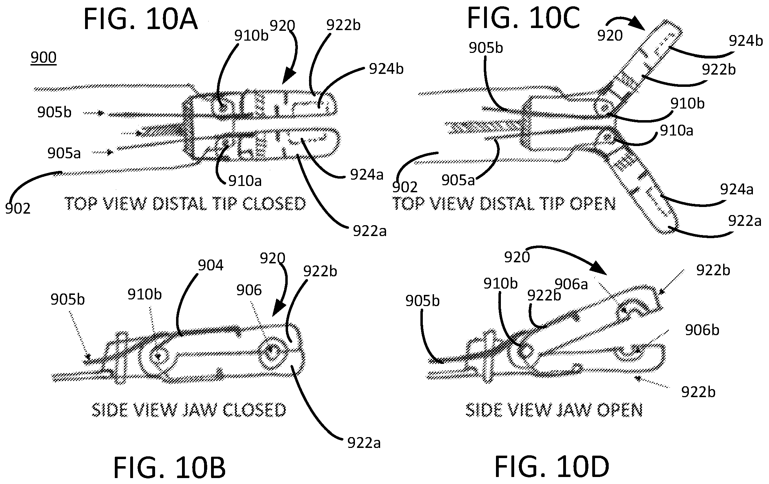

FIG. 10A is a top view and FIG. 10B is a side view of an anastomosis apparatus 900 including a biasing member in a second (closed) configuration, according to an embodiment. FIG. 10C is a top view and FIG. 10D is a side view of the apparatus 900 of FIG. 10A in a first (open) configuration. The apparatus 900 includes an end effector 920 including clamp arms 922a/b defining receptacles 924a/b configured to removably receive coupler rings (e.g., the coupler rings 330a/b). The clamp arms 922a/b may be provided with independent closure controls 905a and 905b, respectively (e.g., ropes, strings, wires, tethers, rods, etc.). Each of the clamp arms 922a/b are coupled to a shaft assembly 902 at pivot joints 910a/b such that the clamp arms 922a/b open like scissors. In some embodiments, the clamp arms may be loaded with a biasing member 904 (e.g., spring loaded) to aid their return to the closed configuration, and that may be actuated via closure controls 905a/b. Such a configuration may allow for exposure of the inner lumen 906 (FIG. 10B) of a dedicated vessel implant split into two (906a and 906b as shown in FIG. 10D) on both sides of each clamp arm 922a/b.

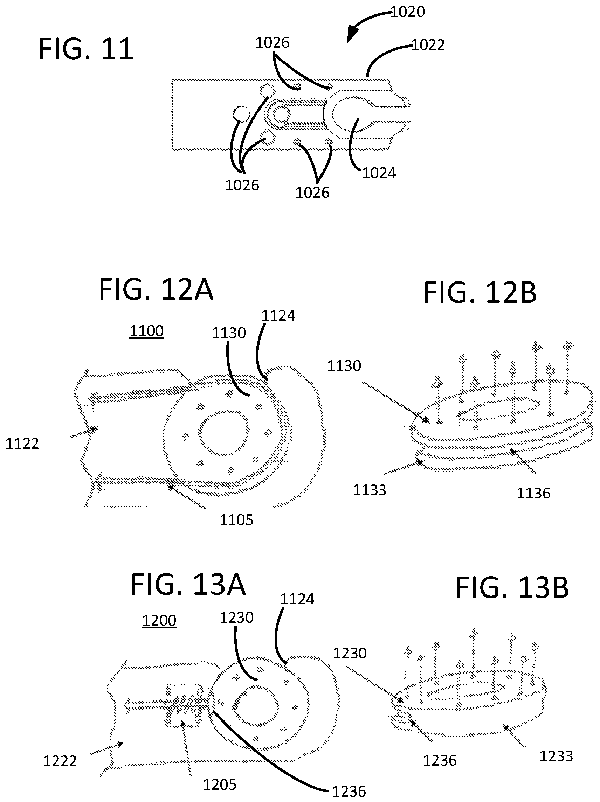

FIG. 11 is a side view of an end effector 1020 that may be included in an anastomosis apparatus (e.g., the apparatus 100, 300), according to an embodiment. The end effector 1020 includes a clamp arms 1022, each defining a receptacle 1024. The clamps arms 1022 may include one or more alignment features 1026 configured for precisely aligning clamp arms 1022 during coupler ring coaptation. Alignment features 1026 may include a plurality of notches, lips and grooves, or other mating features that help with coarse and fine alignment of the coupler rings during actuation of an associated surgical instrument (e.g., the apparatus 100, 300). End effector 1020 may additionally, or alternatively include one or more grips, grooves, surface modifications, latching mechanisms, or any combination thereof to facilitate controlled anastomosis.

FIG. 12A is a side view of a portion of an anastomosis apparatus 1100 that includes a securement mechanism 1105, and FIG. 12B is a side perspective view of a coupler ring 1130 that the securement mechanism 1105 may secure, according to an embodiment. The coupler ring 1130 is disposed in a receptacle 1124 of a clamp arm 1122, and defines a dedicated track or groove 1136 on its outer circumferential side 1133 that allows for proper and secure engagement of a securement mechanism 1105 (e.g., a wire, a thread, a rope, etc.) with the end effector 1122 of a surgical tool. The track 1136 may be friction fit to the securement mechanism 1105 such that once the coupler ring 1130 is ready for deployment, the securement mechanism 1105 can be released, for example, by being pulled from one of its ends. The coupler ring 1130 (or any of the coupler rings described herein) may be formed of any suitable biocompatible material, including, but not limited to, metals, alloys, plastics, ceramics, polymers, or any combination thereof.

FIG. 13A is a side view of a portion of an anastomosis apparatus 1200 that includes a securement mechanism 1205, and FIG. 13B is a side perspective view of a coupler ring 1230 that the securement mechanism 1205 may secure, according to an embodiment. The coupler ring 1205 is removably disposed within a receptacle 1124 of a clamp arm 1122. The coupler ring 1230 defines a dedicated indent 1236 on its circumferential outer side 1233 that may act as a keyhole. Moreover, the clamp arm 1222 may include a securement mechanism 1205 that may include a spring-loaded rod or piston that may be engaged by a user to selectively engage/disengage with the coupler ring 1205 securely and controllably.

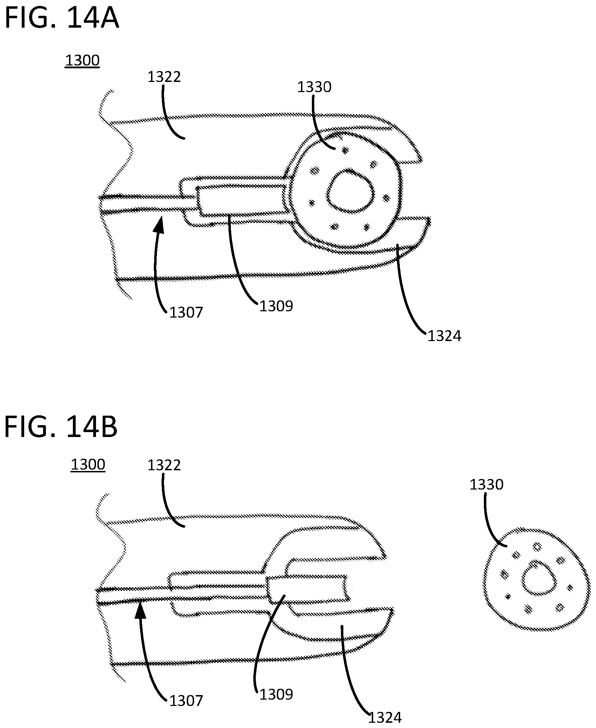

FIG. 14A is a side view of a portion of an anastomosis apparatus 1300 that includes a release mechanism 1307 in a first configuration, according to an embodiment. FIG. 14B is a side view of the portion of the anastomosis apparatus 1300 with the release mechanism 1300 in a second configuration. The apparatus 1300 includes an end effector including a clamp arm 1322 defining a receptacle 1324 within which a coupler ring 1330 is disposed. The release mechanism includes a piston 1309 that may be configured to be selectively pushed out of the clamp arm 1322 into the receptacle to expel the coupler ring 1330 out of the receptacle 1324.

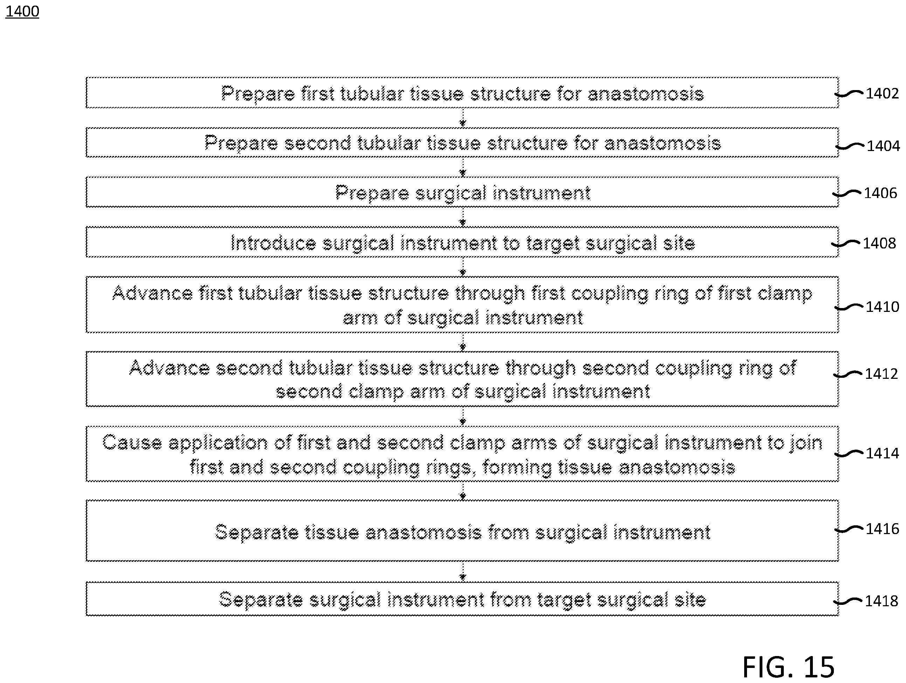

FIG. 15 is a schematic flow chart of a method 1400 (which does not fall within the scope of the claims) for coupling a first vessel to a second vessel using an anastomosis apparatus, according to an embodiment. In some embodiments, the method 1400 includes preparing a first tubular tissue structure for anastomosis, at 1402. At 1404, a second tubular tissue structure may be prepared for anastomosis. At 1406, a surgical instrument (e.g., the apparatus 100, 300 or any other apparatus described herein) is prepared (e.g., loaded with coupler rings 130a/b, 330a/b and moved into intermediate position, as described herein).

At 1408, the surgical instrument is introduced into a surgical site. At 1410, at least a portion of the first tubular tissue structure is advanced or passed through a first distal opening of a first clamp arm of the surgical instrument. At 1412, at least a portion of a second tubular tissue structure is passed through a second distal opening of a second clamp arm of the surgical instrument. At 1414, a change in the configuration of the first and second clamp arms may be caused to securely join the first and second tubular tissue structures. At 1416, the method 1440 may include causing a configuration change of the end effector to separate the first and second tubular tissue structures from the distal aspect of the surgical instrument. At 1418, the surgical instrument is separated from the target surgical site.

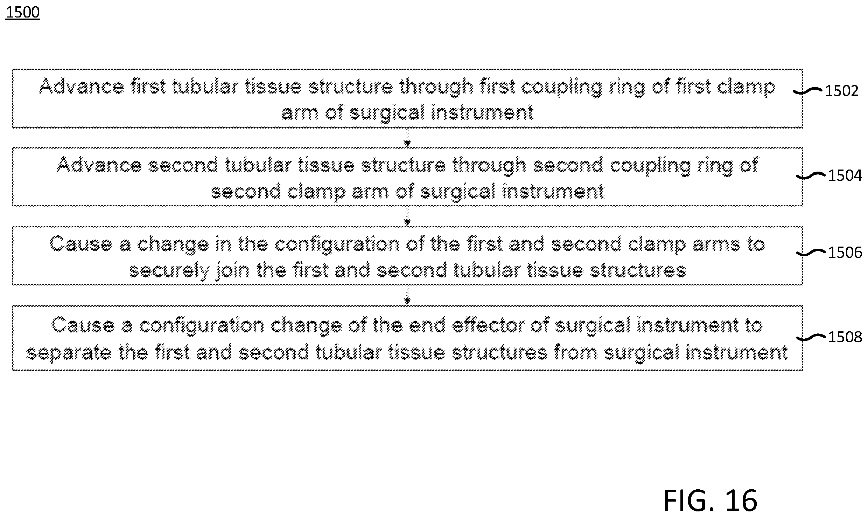

FIG. 16 is a schematic flow chart of a method 1500 for anastomosing a first tubular tissue structure to a second tubular tissue structure, according to an embodiment. The method 1500 may include after preparing a first and second tubular tissue structure for anastomosis, preparing a surgical instrument, and introducing the surgical instrument to a target surgical site, passing at least a portion of first tubular tissue structure through a first coupler ring of a first clamp arm of the surgical instrument, at 1502. At 1504, at least a portion of a second tubular tissue structure is passed through a second coupler ring of a second clamp arm of the surgical instrument. At 1506, a change in the configuration of the first and second clamp arms of the surgical instrument is caused to join the first and second coupler rings. After allowing tissue anastomosis, the method 1500 may also include separating the first and second tubular tissue structures from the surgical instrument, at 1508. The surgical instrument may be separated from the target surgical site.

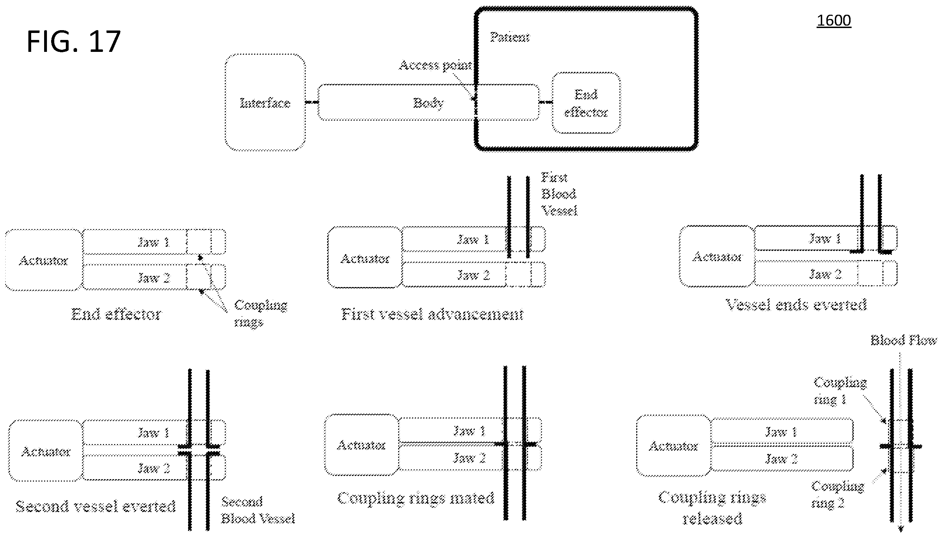

FIG. 17 are schematic illustrations of various operations of a method 1600 for treating BPH, according to an embodiment. The method 1600 may include dissecting tissue to isolate a first tubular tissue structure. A first tubular tissue structure is ligated. The tissue is dissected to isolate a second tubular tissue structure. A second tubular tissue structure is ligated. The method 1600 may also include closing at least a portion of the first and second tubular tissue structure to prevent blood loss. A surgical instrument is introduced to a target surgical site through a tissue access port (such as an incision or laparoscopic trocar).

A change in the configuration of the first and second clamp arms is caused to move the clamps arms into an open configuration. The method 1600 may also include passing at least a portion of first tubular tissue structure through a first coupler ring of a first clamp arm of the surgical instrument, and passing at least a portion of a second tubular tissue structure through a second coupler ring of a second clamp arm of the surgical instrument. The method 1600 may also include securing at least a portion of the first and second tubular tissue structure to at least a portion of the first and second clamp arms; causing a change in the configuration of the first and second clamp arms to securely join the first and second tubular tissue structures. A configuration change of the end effector of the surgical instrument may be caused to separate the first and second tubular tissue structures from the distal aspect of said surgical instrument. A change in the configuration of the first and second clamp arms may be caused to move the clamp arms into a closed configuration, and the surgical instrument may be separated from the target surgical site. The first and second tubular tissue structure may be opened to enable fluid flow therebetween.

Complete closure of the clamp arms of any of the end effectors described herein may be desirable, for example because incomplete closure may lead to potential leakages, clotting, and hemorrhaging. The clamp arms may not only have to mate with each other, but may also have to overcome the forces of friction involved with mating of the coupler rings for example, rings ones where the pins need to slide into corresponding holes. The closure forces may be increased by addition of elements to the end effectors. Such elements may include, for example, a sheath (e.g., the sheath 401) that is be used for external compression of clamp arms together. In some embodiments, mechanical linkages or pull wires internal to the clamp arms may be used to pull the two clamp arms closer to one another. In some embodiments, suitable elements may include magnets, electromagnets, compression using pneumatic, and/or hydraulic means. Additionally, or alternatively, the clamp arms may include ultrasound transducers that help overcome the frictional forces as the pins of the coupler rings slide into the corresponding snap-fit channels.

In some embodiments, any of the end effectors described herein may be configured to articulate about the distal end of the elongate body or shaft assembly by an angle in a range of at least about 20 degrees (e.g., 20, 40, 60, 80, 90, 100, 110, or 120 degrees, inclusive). This may increase the visibility of the various elements critical to performing an anastomosis procedure.

As used herein, the singular forms "a", "an" and "the" include plural referents unless the context clearly dictates otherwise. Thus, for example, the term "a member" is intended to mean a single member or a combination of members, "a material" is intended to mean one or more materials, or a combination thereof.

As used herein, the terms "about" and "approximately" generally mean plus or minus 10% of the stated value. For example, about 0.5 would include 0.45 and 0.55, about 10 would include 9 to 11, about 1000 would include 900 to 1100.

As utilized herein, the terms "substantially" and similar terms are intended to have a broad meaning in harmony with the common and accepted usage by those of ordinary skill in the art to which the subject matter of this disclosure pertains. For example, the term "substantially flat" would mean that there may be *de minimis* amount of surface variations or undulations present due to manufacturing variations present on an otherwise flat surface. It should be understood by those of skill in the art who review this disclosure that these terms are intended to allow a description of certain features described and claimed without restricting the scope of these features to the precise arrangements and /or numerical ranges provided. Accordingly, these terms should be interpreted as indicating that insubstantial or inconsequential modifications or alterations of the subject matter described and claimed are considered to be within the scope of the inventions as recited in the appended claims.

It should be noted that the term "exemplary" as used herein to describe various embodiments is intended to indicate that such embodiments are possible examples, representations, and/or illustrations of possible embodiments (and such term is not intended to connote that such embodiments are necessarily extraordinary or superlative examples).

The terms "coupled," and the like as used herein mean the joining of two members directly or indirectly to one another. Such joining may be stationary (e.g., permanent) or moveable (e.g., removable, or releasable). Such joining may be achieved with the two members or the two members and any additional intermediate members being integrally formed as a single unitary body with one another or with the two members or the two members and any additional intermediate members being attached to one another.

It is important to note that the construction and arrangement of the various exemplary embodiments are illustrative only. Although only a few embodiments have been described in detail in this disclosure, those skilled in the art who review this disclosure will readily appreciate that many modifications are possible (e.g., variations in sizes, dimensions, structures, shapes and proportions of the various elements, values of parameters, mounting arrangements, use of materials, colors, orientations, etc.) without materially departing from the novel teachings and advantages of the subject matter described herein. Other substitutions, modifications, changes, and omissions may also be made in the design, operating conditions, and arrangement of the various exemplary embodiments without departing from the scope of the present invention, as characterized by the appended claims.

While this specification contains many specific implementation details, these should not be construed as limitations on the scope of any inventions or of what may be claimed, but rather as descriptions of features specific to particular implementations of particular inventions. Certain features described in this specification in the context of separate implementations can also be implemented in combination in a single implementation. Conversely, various features described in the context of a single implementation can also be implemented in multiple implementations separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

Thus, particular implementations of the invention have been described. Other implementations are within the scope of the following claims. In some cases, the actions recited in the claims can be performed in a different order and still achieve desirable results. In addition, the processes depicted in the accompanying figures do not necessarily require the particular order shown, or sequential order, to achieve desirable results. In certain implementations, multitasking and parallel processing may be advantageous.

## Claims

1. An apparatus (100), comprising:
an interface (104);
an elongate body (102) coupled to the interface and extending longitudinally therefrom; and
an end effector (120) coupled to a distal end of the elongate body, the end effector including:
a first end effector element (122a) defining a first receptacle structured to hold a first coupler element (130a) of a coupler (130), the first coupler element configured to receive an axial end of a first vessel, and
a second end effector element (122b) defining a second receptacle structured to hold a second coupler element (130b) of the coupler, the second coupler element configured to receive an axial end of a second vessel,
wherein the end effector is configured to move between a first configuration in which at least a portion of the first end effector element is separate from a corresponding portion of the second end effector element such that the first coupler element is separate from the second coupler element, and a second configuration in which at least the portion of the first end effector element is proximate to the corresponding portion of the second end effector element such that the first coupler element is coupled to the second coupler element so as to couple the first vessel to the second vessel, **characterized in that** the apparatus further comprises a sheath (10) disposed around the elongate body and configured to be displaced axially towards the end effector to urge the end effector into the second configuration.

2. The apparatus of claim 1, wherein:
proximal ends of the first end effector element and the second end effector element are coupled to a distal end of the elongate body,
the first coupler element and the second coupler element are configured to be removably coupled to corresponding distal ends of the first end effector element and the second effector element, respectively, and
the first and the second end effector elements are configured to articulate about the distal end of the elongate body in a first direction to move the end effector between the first and the second configurations.

3. The apparatus of claim 2, wherein the proximal ends of the first end effector element and the second end effector element are coupled to the distal end of the elongate body via an actuation mechanism structured to allow articulation of the first and second end effector elements.

4. The apparatus of claim 3, wherein the actuation mechanism is configured to allow articulation of the end effector about the distal end of the elongate body in a second direction different from the first direction.

5. The apparatus of claim 3, wherein the actuation mechanism includes a first linkage arm coupled to the first end effector element and a second linkage arm coupled to the second end effector element, each of the first and a second linkage arms including a central hinge such that moving a distal end of each of the first and second linkage arms proximate to a corresponding proximate end of the first and second linkage arms, causes the first and second linkage arms to articulate about their respective central hinge away from each other so as to selectively move the end effector from the first configuration to the second configuration.

6. The apparatus of claim 3, wherein the actuation mechanism includes:
at least one pulley coupled to a corresponding one of the first end effector element or the second end effector element, and
at least one tether coupled to the at least one pulley, the at least one tether configured to be displaced longitudinally to cause the at least one pulley to rotate and move the end effector between the first and the second configurations.

7. The apparatus of claim 3, wherein the actuation mechanism includes:
at least one rod coupled to a corresponding one of the first end effector element or the second end effector element and configured to rotate to cause the end effector to move between the first and second configurations.

8. The apparatus of claim 7, wherein the at least one rod is coupled to the corresponding one of the first end effector element or the second end effector element proximate to a radially outer edge of the corresponding one of the first end effector element or the second end effector element.

9. The apparatus of any one of claims 1 to 8, further including at least one closure element coupled to the first end effector element and the second end effector element and configured to urge the end effector towards the second configuration.

10. The apparatus of any of the preceding claims, wherein each of first end effector element and the second end effector element are configured to torsionally bend along their respective axis to move between the first configuration and the second configuration.

11. The apparatus of any of the preceding claims, wherein each of the first end effector element and the second end effector element include alignment features defined on corresponding surfaces thereof, the alignment features configured to align the first and the second end effector elements as the end effector moves into the second configuration to facilitate alignment of the first coupler element with the second coupler element.

12. The apparatus of any one of the preceding claims, further comprising:
a securement mechanism operatively coupled to the end effector and configured to secure at least one of the first coupler element or the second coupler element in a first configuration of the end effector.

13. The apparatus of claim 12, wherein the securement mechanism is configured to be selectively actuated to release the coupler from the end effector in the second configuration.

14. The apparatus of claim 12, further comprising:
a release mechanism configured to selectively urge the first coupler element and/or the second coupler element out of the end effector.

## Patentansprüche

1. Vorrichtung (100), umfassend:
eine Schnittstelle (104);
einen länglichen Körper (102), der mit der Schnittstelle gekoppelt ist und sich in Längsrichtung davon erstreckt; und
einen Endeffektor (120), der mit einem distalen Ende des länglichen Körpers gekoppelt ist, wobei der Endeffektor aufweist:
ein erstes Endeffektorelement (122a), das eine erste Aufnahme definiert, die strukturiert ist, um ein erstes Kopplungselement (130a) eines Kopplers (130) zu halten,
wobei das erste Kopplungselement dazu ausgelegt ist, ein axiales Ende eines ersten Gefäßes aufzunehmen, und
ein zweites Endeffektorelement (122b), das eine zweite Aufnahme definiert, die strukturiert ist, um ein zweites Kopplungselement (130b) des Kopplers zu halten, wobei das zweite Kopplungselement dazu ausgelegt ist, ein axiales Ende eines zweiten Gefäßes aufzunehmen,
wobei der Endeffektor dazu ausgelegt ist, sich zwischen einer ersten Auslegung, in der mindestens ein Abschnitt des ersten Endeffektorelements von einem entsprechenden Abschnitt des zweiten Endeffektorelements getrennt ist, so dass das erste Kopplungselement von dem zweiten Kopplungselement getrennt ist, und einer zweiten Auslegung, in der mindestens der Abschnitt des ersten Endeffektorelements in der Nähe des entsprechenden Abschnitts des zweiten Endeffektorelements liegt, so dass das erste Kopplungselement mit dem zweiten Kopplungselement gekoppelt ist, um das erste Gefäß mit dem zweiten Gefäß zu koppeln, zu bewegen, **dadurch gekennzeichnet, dass** die Vorrichtung ferner eine Hülse (10) umfasst, die um den länglichen Körper herum angeordnet und dazu ausgelegt ist, in Richtung des Endeffektors axial verschoben zu werden, um den Endeffektor in die zweite Auslegung zu drängen.

2. Vorrichtung nach Anspruch 1, wobei:
die proximalen Enden des ersten Endeffektorelements und des zweiten Endeffektorelements mit einem distalen Ende des länglichen Körpers gekoppelt sind,
das erste Kopplungselement und das zweite Kopplungselement dazu ausgelegt sind, entfernbar mit den entsprechenden distalen Enden des ersten Endeffektorelements bzw. des zweiten Endeffektorelements gekoppelt zu werden, und
das erste und das zweite Endeffektorelement dazu ausgelegt sind, um das distale Ende des länglichen Körpers in eine erste Richtung auszulenken, um den Endeffektor zwischen der ersten und der zweiten Auslegung zu bewegen.

3. Vorrichtung nach Anspruch 2, wobei die proximalen Enden des ersten Endeffektorelements und des zweiten Endeffektorelements über einen Betätigungsmechanismus mit dem distalen Ende des länglichen Körpers gekoppelt sind, der strukturiert ist, um die Auslenkung des ersten und des zweiten Endeffektorelements zu ermöglichen.

4. Vorrichtung nach Anspruch 3, wobei der Betätigungsmechanismus dazu ausgelegt ist, die Auslenkung des Endeffektors um das distale Ende des länglichen Körpers in eine zweite Richtung, die sich von der ersten Richtung unterscheidet, zu ermöglichen.

5. Vorrichtung nach Anspruch 3, wobei der Betätigungsmechanismus einen ersten Gelenkarm, der mit dem ersten Endeffektorelement gekoppelt ist, und einen zweiten Gelenkarm, der mit dem zweiten Endeffektorelement gekoppelt ist, aufweist, wobei jeder des ersten und eines zweiten Gelenkarms ein zentrales Gelenk aufweist, so dass das Bewegen eines distalen Endes eines jeden des ersten und des zweiten Gelenkarms in der Nähe eines entsprechenden proximalen Endes des ersten und des zweiten Gelenkarms bewirkt, dass der erste und der zweite Gelenkarm um ihr jeweiliges zentrales Gelenk voneinander weg auslenken, um den Endeffektor gezielt aus der ersten Auslegung in die zweite Auslegung zu bewegen.

6. Vorrichtung nach Anspruch 3, wobei der Betätigungsmechanismus aufweist:
mindestens eine Riemenscheibe, die mit einem entsprechenden einen des ersten Endeffektorelements oder des zweiten Endeffektorelements gekoppelt ist, und
mindestens einen Riemen, der mit der mindestens einen Riemenscheibe gekoppelt ist, wobei der mindestens eine Riemen dazu ausgelegt ist, in Längsrichtung verschoben zu werden, um zu bewirken, dass sich die mindestens eine Riemenscheibe dreht und den Endeffektor zwischen der ersten und der zweiten Auslegung bewegt.

7. Vorrichtung nach Anspruch 3, wobei der Betätigungsmechanismus aufweist:
mindestens eine Stange, die mit einem entsprechenden einen des ersten Endeffektorelements oder des zweiten Endeffektorelements gekoppelt und dazu ausgelegt ist, sich zu drehen, um zu bewirken, dass sich der Endeffektor zwischen der ersten und der zweiten Auslegung bewegt.

8. Vorrichtung nach Anspruch 7, wobei die mindestens eine Stange mit dem entsprechenden einen des ersten Endeffektorelements oder des zweiten Endeffektorelements in der Nähe eines radial äußeren Randes des entsprechenden einen des ersten Endeffektorelements oder des zweiten Endeffektorelements gekoppelt ist.

9. Vorrichtung nach einem der Ansprüche 1 bis 8, ferner aufweisend mindestens ein Schließelement, das mit dem ersten Endeffektorelement und dem zweiten Endeffektorelement gekoppelt und dazu ausgelegt ist, den Endeffektor in Richtung der zweiten Auslegung zu drängen.

10. Vorrichtung nach einem der vorhergehenden Ansprüche, wobei jedes des ersten Endeffektorelements und des zweiten Endeffektorelements dazu ausgelegt ist, sich entlang seiner Achse drehelastisch zu biegen, um sich zwischen der ersten Auslegung und der zweiten Auslegung zu bewegen.

11. Vorrichtung nach einem der vorhergehenden Ansprüche, wobei jedes des ersten Endeffektorelements und des zweiten Endeffektorelements Ausrichtmerkmale aufweist, die an entsprechenden Oberflächen davon definiert sind, wobei die Ausrichtmerkmale dazu ausgelegt sind, das erste und das zweite Endeffektorelement auszurichten, wenn sich der Endeffektor in die zweite Auslegung bewegt, um die Ausrichtung des ersten Kopplungselements auf das zweite Kopplungselement zu erleichtern.

12. Vorrichtung nach einem der vorhergehenden Ansprüche, ferner umfassend:
einen Befestigungsmechanismus, der funktional mit dem Endeffektor gekoppelt und dazu ausgelegt ist, mindestens eins des ersten Kopplungselements oder des zweiten Kopplungselements in einer ersten Auslegung des Endeffektors zu befestigen.

13. Vorrichtung nach Anspruch 12, wobei der Befestigungsmechanismus dazu ausgelegt ist, gezielt betätigt zu werden, um in der zweiten Auslegung den Koppler von dem Endeffektor zu lösen.

14. Vorrichtung nach Anspruch 12, ferner umfassend:
einen Freigabemechanismus, der dazu ausgelegt ist, das erste Kopplungselement und/oder das zweite Kopplungselement gezielt aus dem Endeffektor heraus zu drängen.

## Revendications

1. Appareil (100), comprenant :
une interface (104) ;
un corps allongé (102) couplé à l'interface et s'étendant longitudinalement à partir de celle-ci ; et
un effecteur terminal (120) couplé à une extrémité distale du corps allongé, l'effecteur terminal comportant :
un premier élément d'effecteur terminal (122a) définissant un premier réceptacle structuré pour maintenir un premier élément de coupleur (130a) d'un coupleur (130), le premier élément de coupleur étant configuré pour recevoir une extrémité axiale d'un premier vaisseau, et
un second élément d'effecteur terminal (122b) définissant un second réceptacle structuré pour maintenir un second élément de coupleur (130b) du coupleur, le second élément de coupleur étant configuré pour recevoir une extrémité axiale d'un second vaisseau,
dans lequel l'effecteur terminal est configuré pour se déplacer entre une première configuration dans laquelle au moins une partie du premier élément d'effecteur terminal est distincte d'une partie correspondante du second élément d'effecteur terminal de telle sorte que le premier élément de coupleur soit distinct du second élément de coupleur, et une seconde configuration dans laquelle au moins la partie du premier élément d'effecteur terminal est proche de la partie correspondante du second élément d'effecteur terminal de telle sorte que le premier élément de coupleur soit couplé au second élément de coupleur de manière à coupler le premier vaisseau au second vaisseau, **caractérisé en ce que** l'appareil comprend en outre une gaine (10) disposée autour du corps allongé et configurée pour être déplacée axialement vers l'effecteur terminal pour pousser l'effecteur terminal dans la seconde configuration.

2. Appareil selon la revendication 1, dans lequel :
des extrémités proximales du premier élément d'effecteur terminal et du second élément d'effecteur terminal sont couplées à une extrémité distale du corps allongé,
le premier élément de coupleur et le second élément de coupleur sont configurés pour être couplés de manière amovible aux extrémités distales correspondantes du premier élément d'effecteur terminal et du second élément d'effecteur, respectivement, et
les premier et second éléments d'effecteur terminal sont conçus pour s'articuler autour de l'extrémité distale du corps allongé dans une première direction afin de déplacer l'effecteur terminal entre les première et seconde configurations.

3. Appareil selon la revendication 2, dans lequel les extrémités proximales du premier élément d'effecteur terminal et du second élément d'effecteur terminal sont couplées à l'extrémité distale du corps allongé par l'intermédiaire d'un mécanisme d'actionnement structuré pour permettre l'articulation des premier et second éléments d'effecteur terminal.

4. Appareil selon la revendication 3, dans lequel le mécanisme d'actionnement est configuré pour permettre l'articulation de l'effecteur terminal autour de l'extrémité distale du corps allongé dans une seconde direction différente de la première direction.

5. Appareil selon la revendication 3, dans lequel le mécanisme d'actionnement comporte un premier bras de liaison couplé au premier élément d'effecteur terminal et un second bras de liaison couplé au second élément d'effecteur terminal, chacun des premier et second bras de liaison comportant une charnière centrale de telle sorte que déplacer une extrémité distale de chacun des premier et second bras de liaison à proximité d'une extrémité proximale correspondante des premier et second bras de liaison amène les premier et second bras de liaison à s'articuler autour de leur articulation centrale respective à l'opposé l'un de l'autre de manière à déplacer sélectivement l'effecteur terminal de la première configuration à la seconde configuration.

6. Appareil selon la revendication 3, dans lequel le mécanisme d'actionnement comporte :
au moins une poulie accouplée à un élément correspondant parmi le premier élément d'effecteur terminal et le second élément d'effecteur terminal, et
au moins un câble d'attache couplé à l'au moins une poulie, l'au moins un câble d'attache étant configuré pour être déplacé longitudinalement pour amener l'au moins une poulie à tourner et déplacer l'effecteur terminal entre les première et seconde configurations.

7. Appareil selon la revendication 3, dans lequel le mécanisme d'actionnement comporte :
au moins une tige couplée à un élément de correspondant parmi le premier élément d'effecteur terminal et le second élément d'effecteur terminal et configurée pour tourner de manière à amener l'effecteur terminal à se déplacer entre les première et seconde configurations.

8. Appareil selon la revendication 7, dans lequel l'au moins une tige est couplée à l'élément correspondant parmi le premier élément d'effecteur terminal et le second élément d'effecteur terminal à proximité d'un bord radialement externe de l'élément correspondant parmi le premier élément d'effecteur terminal et le second élément d'effecteur terminal.

9. Appareil selon l'une quelconque des revendications 1 à 8, comportant en outre au moins un élément de fermeture couplé au premier élément d'effecteur terminal et au second élément d'effecteur terminal et configuré pour pousser l'effecteur terminal vers la seconde configuration.

10. Appareil selon l'une quelconque des revendications précédentes, dans lequel chacun du premier élément d'effecteur terminal et du second élément d'effecteur terminal est configuré pour se courber en torsion le long de son axe respectif pour se déplacer entre la première configuration et la seconde configuration.

11. Appareil selon l'une quelconque des revendications précédentes, dans lequel chacun du premier élément d'effecteur terminal et du second élément d'effecteur terminal comporte des caractéristiques d'alignement définies sur des surfaces correspondantes de ceux-ci, les caractéristiques d'alignement étant configurées pour aligner les premier et second éléments d'effecteur terminal lorsque l'effecteur terminal se déplace dans la seconde configuration pour faciliter l'alignement du premier élément de coupleur sur le second élément de coupleur.

12. Appareil selon l'une quelconque des revendications précédentes, comprenant en outre :
un mécanisme de fixation couplé fonctionnellement à l'effecteur terminal et configuré pour fixer au moins un élément parmi le premier élément de coupleur et le second élément de coupleur dans une première configuration de l'effecteur terminal.

13. Appareil selon la revendication 12, dans lequel le mécanisme de fixation est configuré pour être actionné sélectivement pour libérer le coupleur de l'effecteur terminal dans la seconde configuration.

14. Appareil selon la revendication 12, comprenant en outre :
un mécanisme de libération conçu pour pousser sélectivement le premier élément de coupleur et/ou le second élément de coupleur hors de l'effecteur terminal.
